(19) Europäisches Patentamt
European Patent Office
Office européen des brevets

(11) EP 4 414 357 A1

(12) EUROPEAN PATENT APPLICATION
published in accordance with Art. 153(4) EPC

(43) Date of publication:
**14.08.2024 Bulletin 2024/33**

(21) Application number: **22878549.9**

(22) Date of filing: **05.10.2022**

(51) International Patent Classification (IPC):
***C07C 68/08*** (2006.01) ***B01D 3/14*** (2006.01)
***C07C 68/06*** (2020.01) ***C07C 69/96*** (2006.01)

(52) Cooperative Patent Classification (CPC):
**B01D 3/14; C07C 68/06; C07C 68/08; C07C 69/96;**
Y02P 20/10

(86) International application number:
**PCT/JP2022/037264**

(87) International publication number:
**WO 2023/058681 (13.04.2023 Gazette 2023/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.10.2021 JP 2021163793**

(71) Applicant: **Asahi Kasei Kabushiki Kaisha**
**Tokyo 1000006 (JP)**

(72) Inventor: **OCHI, Hiroyuki**
**Tokyo 100-0006 (JP)**

(74) Representative: **Strehl Schübel-Hopf & Partner**
**Maximilianstrasse 54**
**80538 München (DE)**

# (54) METHOD FOR PRODUCING HIGH-PURITY DIARYL CARBONATE

(57) Provided is a method for industrially producing high-purity diaryl carbonate, comprising: a first purification step of continuously introducing a mixture containing diaryl carbonate to a purifying column A having a side cut discharge port, and continuously separating by distillation the mixture into a column top component (At), a side cut component (As) containing diaryl carbonate, and a column bottom component (Ab) containing a catalyst; and a second purification step of continuously introducing the side cut component (As) to a diaryl carbonate purifying column B having a side cut discharge port, and continuously separating by distillation the component into three components, a column top component (Bt), a side cut component (Bs), and a column bottom component (Bb), thereby obtaining high-purity diaryl carbonate as the side cut component (Bs).

[Figure 1]

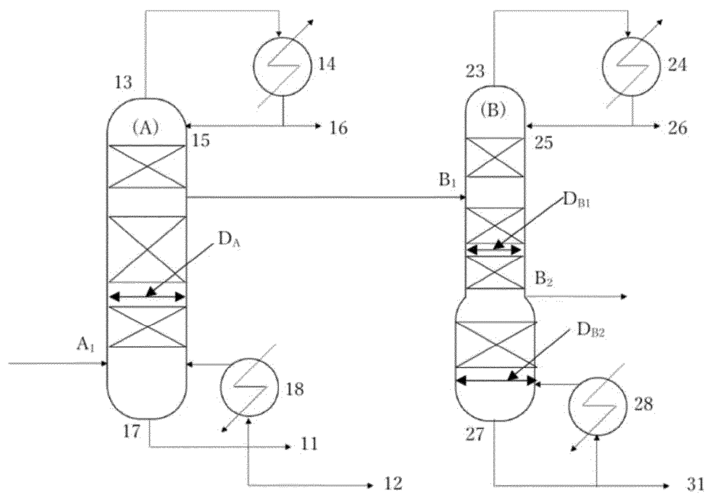




Processed by Luminess, 75001 PARIS (FR)

## Description

Technical Field

The present invention relates to a method for producing high-purity diaryl carbonate.

Background Art

For example, the production of aromatic polycarbonate at an industrial scale through transesterification reaction requires obtaining a large amount of high-purity diaryl carbonate at an industrial scale. Such a large amount of high-purity diaryl carbonate is difficult to obtain at an industrial scale. Thus, there is a demand for the development of a useful method for producing high-purity diaryl carbonate. As for the high-purity diaryl carbonate, for example, Patent Documents 1 and 2 disclose a production method.

List of Prior Art Documents

Patent Document

Patent Document 1: International Publication No. WO 2006/025478
Patent Document 2: International Publication No. 2007/072705

Summary of Invention

Problems to be Solved by Invention

However, conventional techniques, particularly, methods using a conventional catalyst separation column and diaryl carbonate purifying column, such as the methods described in Patent Documents 1 and 2, require evaporating alkyl aryl carbonate twice and require a large amount of energy for purification and separation. Furthermore, use of a large amount of energy causes alteration of alkyl aryl carbonate or diaryl carbonate ascribable to purification and a large amount of loss ascribable to distillation. Thus, these techniques or methods are susceptible to improvement.

Accordingly, an object of the present invention is to provide a method for producing high-purity diaryl carbonate which can reduce an energy usage in purification and reduce the amount of loss ascribable to distillation.

Means for Solving Problems

The present inventor has conducted studies to find a specific method that can attain the object described above, and consequently reached the present invention. Specifically, the present invention is as follows.

[1] A method for continuously producing high-purity diaryl carbonate, the method for industrially producing high-purity diaryl carbonate and comprising:

a first purification step of continuously introducing a mixture containing diaryl carbonate to a purifying column A having a side cut discharge port, and continuously separating by distillation the mixture into a column top component (At), a side cut component (As) containing diaryl carbonate, and a column bottom component (Ab) containing a catalyst; and

a second purification step of continuously introducing the side cut component (As) to a diaryl carbonate purifying column B having a side cut discharge port, and continuously separating by distillation the component into three components, a column top component (Bt), a side cut component (Bs), and a column bottom component (Bb), thereby obtaining high-purity diaryl carbonate as the side cut component (Bs).

[2] The production method according to [1], wherein the mixture contains the diaryl carbonate at a ratio of 40% by mass or more and 90% by mass or less.

[3] The production method according to [1] or [2], wherein the mixture contains alkyl aryl carbonate at a ratio of 10% by mass or more and 50% by mass or less.

[4] The production method according to any of [1] to [3], wherein a content of the diaryl carbonate in the side cut component (As) is 80% by mass or more, and a content of the diaryl carbonate in the side cut component (Bs) is

99.1% by mass or more.

[5] The production method according to any of [1] to [4], wherein the theoretical number of stages An from the column bottom to the side cut discharge port of the purifying column A is in a range of 10 or more and 19 or less.

[6] The production method according to any of [1] to [5], wherein a ratio (SA/BsF) of column cross section (SA ($m^2$)) of the purifying column A to a production amount (BsF (ton/hr)) as the discharged side cut component (Bs) is in a range of 0.5 or more and 5 or less.

[7] The production method according to any of [1] to [6], wherein a ratio (DB1/DB2) of an upper column diameter (DB1 (m)) to a lower column diameter (DB2 (m)) in the purifying column B is in a range of 0.1 or more and 0.9 or less.

[8] The production method according to any of [1] to [7], wherein the purifying column A comprises at least one reboiler selected from the group consisting of forced circulation type, cross pipe falling film type, and thin-film evaporation type.

[9] The production method according to any of [1] to [8], wherein

a packing of the purifying column A and/or the purifying column B is a structured packing, wherein
the structured packing comprises at least one selected from the group consisting of Mellapak, Gempak, Techno Pack, Flexipac, Sulzer Packings, Goodloe Packing, Glitsch Grid, and a gauze packing.

[10] The production method according to any of [1] to [9], wherein the mixture is a reaction mixture obtained through at least one reaction selected from the group consisting of (i) transesterification reaction of dialkyl carbonate and aryl alcohol, (ii) disproportionation reaction of alkyl aryl carbonate, and (iii) transesterification reaction of alkyl aryl carbonate and aryl alcohol, in the presence of a catalyst.

[11] The production method according to any of [1] to [10], further comprising the steps of:

continuously supplying dialkyl carbonate and aryl alcohol into a first continuous multistage distillation column containing a catalyst, simultaneously performing a reaction and distillation in the first continuous multistage distillation column, continuously discharging a first column low-boiling reaction mixture containing alcohols thus formed in a gas form from an upper portion of the first continuous multistage distillation column, and continuously discharging a first column high-boiling reaction mixture containing alkyl aryl carbonate thus formed in a liquid form from a lower portion of the first continuous multistage distillation column to continuously produce alkyl aryl carbonate; and
continuously supplying the first column high-boiling reaction mixture into a second continuous multistage distillation column containing a catalyst, simultaneously performing a reaction and distillation in the second continuous multistage distillation column, continuously discharging a second column low-boiling reaction mixture containing dialkyl carbonates thus formed in a gas form from an upper portion of the second continuous multistage distillation column, and continuously discharging a second column high-boiling reaction mixture containing diaryl carbonate thus formed in a liquid form from a lower portion of the second continuous multistage distillation column to continuously produce diaryl carbonate, wherein
the mixture is the second column high-boiling reaction mixture containing diaryl carbonate.

[12] The production method according to any of [1] to [11], wherein the diaryl carbonate is diphenyl carbonate.

Advantages of Invention

The present invention can provide a method for producing high-purity diaryl carbonate which can reduce an energy usage in purification and reduce the amount of loss ascribable to distillation.

Brief Description of Drawings

[Figure 1] Figure 1 is a schematic view showing one example of an apparatus comprising a purifying column A connected with a diphenyl carbonate purifying column B used in the present invention.

[Figure 2] Figure 2 is a schematic view showing one example of an apparatus comprising a purifying column A connected with a diphenyl carbonate purifying column B in a conventional technique.

[Figure 3] Figure 3 is a schematic view showing one example of a continuous multistage distillation column $T_0$. An internal formed from a sieve tray is installed in the inside of a body.

[Figure 4] Figure 4 is a schematic view showing one example of a first continuous multistage distillation column. An internal is installed in the inside of a body.

[Figure 5] Figure 5 is a schematic view showing one example of a second continuous multistage distillation column. A structured packing is installed in an upper portion, and an internal formed from a sieve tray is installed in a lower portion, in the inside of a body.

[Figure 6] Figure 6 is a schematic view showing one example of an apparatus comprising a first continuous multistage distillation column connected with a second continuous multistage distillation column.

Mode for Carrying Out Invention

Hereinafter, the mode for carrying out the present invention (hereinafter, referred to as the "present embodiment") will be described in detail with reference to the drawings, if necessary. However, the present invention is not limited thereby, and various changes or modifications can be made therein without departing from the spirit of the present invention. In the drawings, positional relationships indicated by terms such as "up", "down", "right", and "left" are based on the positional relationships shown in the drawings, unless otherwise specified. Dimensional ratios in the drawings are not limited to the shown ratios.

The method for industrially producing high-purity diaryl carbonate according to the present embodiment is a method for continuously producing high-purity diaryl carbonate, comprising:

a first purification step of continuously introducing a mixture containing diaryl carbonate to a purifying column A having a side cut discharge port, and continuously separating by distillation the mixture into a column top component (At), a side cut component (As) containing diaryl carbonate, and a column bottom component (Ab) containing a catalyst; and

a second purification step of continuously introducing the side cut component (As) to a diaryl carbonate purifying column B having a side cut discharge port, and continuously separating by distillation the component into three components, a column top component (Bt), a side cut component (Bs), and a column bottom component (Bb), thereby obtaining high-purity diaryl carbonate as the side cut component (Bs).

Owing to the features as described above, the method for industrially producing high-purity diaryl carbonate according to the present embodiment exerts effects of being able to reduce energy usage in purification and reduce the amount of loss ascribable to distillation. Although a mechanism under which such effects are exerted is not certain, the present inventor deduces the following mechanism.

The side cut discharge port is installed in the purifying column A, whereby a large proportion of alkyl aryl carbonate can be separated by the purifying column A. This can suppress the alteration of alkyl aryl carbonate or diaryl carbonate while decreasing a load on the diaryl carbonate purifying column B. As a result, the amount of loss of diaryl carbonate ascribable to distillation can be reduced. Such reduction in the amount of loss of diaryl carbonate can decrease alteration product waste, can further reduce an energy usage with respect to a production amount, and is presumably capable of reducing an environmental load in the production of high-purity diaryl carbonate.

The method for industrially producing high-purity diaryl carbonate according to the present embodiment can reduce an energy usage in purification, can also lower the concentration of an altered substance (hereinafter, also referred to as "XA") in diphenyl carbonate, and is capable of preventing deterioration in the quality of diaryl carbonate ascribable to an altered substance.

In the method for industrially producing high-purity diaryl carbonate according to the present embodiment, the mixture to be introduced to the purifying column A preferably contains the diaryl carbonate at a ratio of 40% by mass or more and 90% by mass or less, more preferably at a ratio of 45% by mass or more and 85% by mass or less, further preferably at a ratio of 50% by mass or more and 80% by mass or less. When the content of the diaryl carbonate in the mixture to be introduced to the purifying column A is equal to or more than the lower limit value described above, loss tends to be able to be reduced. When the content is equal to or less than the upper limit value described above, poor control tends to be able to be prevented.

In the method for industrially producing high-purity diaryl carbonate according to the present embodiment, the mixture to be introduced to the purifying column A preferably contains alkyl aryl carbonate at a ratio of 10% by mass or more and 50% by mass or less, more preferably at a ratio of 15% by mass or more and 45% by mass or less, further preferably at a ratio of 20% by mass or more and 40% by mass or less. When the content of the alkyl aryl carbonate in the mixture to be introduced to the purifying column A is equal to or more than the lower limit value described above, poor control tends to be able to be prevented. When the content is equal to or less than the upper limit value described above, loss tends to be able to be reduced.

In the present embodiment, the composition of the mixture to be introduced to the purifying column A can be measured by a method described in Examples mentioned later.

In the method for industrially producing high-purity diaryl carbonate according to the present embodiment, the mixture to be introduced to the purifying column A is not particularly limited. The mixture and an apparatus and a method

for producing the mixture particularly preferably involve diphenyl carbonate produced and purified by reacting ethylene oxide with $CO_2$, reacting ethylene carbonate thus produced and purified with methanol, and subjecting dimethyl carbonate thus produced and purified and purified phenol to a reactive distillation method. Such diphenyl carbonate is an ultra-high-purity product free from an alkali metal/alkaline earth metal and chlorine. Such a production method is not particularly limited. See, for example, International Publication Nos. WO 2004/105696, WO 2007/34669, WO 2007/60893, WO 2007/60984, WO 2007/69462, WO 2007/69513, WO 2007/69514, WO 2007/72728, WO 2005/123638, WO 2007/74692, WO 2007/88782, WO 2006/1256, WO 2006/1257, WO 2006/6566, WO 2006/6568, WO 2006/6585, WO 2006/6588, WO 2006/41075, WO 2006/43491, WO 2007/69529, WO 2007/69531, and WO 2007/72705. In addition, a diphenyl carbonate mixture produced by reacting an alcohol with $CO_2$, and reacting dialkyl carbonate thus produced and purified with purified phenol is also suitable. Such a production method is not particularly limited. See, for example, International Publication Nos. WO 2005/783, WO 2007/114130, WO 2003/55840, WO 2011/105442, and WO 2006/67982. The mixture is preferably, for example, a reaction mixture obtained through at least one reaction selected from the group consisting of (i) transesterification reaction of dialkyl carbonate and aryl alcohol, (ii) disproportionation reaction of alkyl aryl carbonate, and (iii) transesterification reaction of alkyl aryl carbonate and aryl alcohol, in the presence of a catalyst. When the mixture to be introduced to the purifying column A is such a reaction mixture, reduction in loss and the prevention of poor control tend to be attained.

The method for industrially producing high-purity diaryl carbonate according to the present embodiment comprises:

a first purification step of continuously introducing a mixture containing diaryl carbonate to a purifying column A having a side cut discharge port, and continuously separating by distillation the mixture into a column top component (At), a side cut component (As) containing diaryl carbonate, and a column bottom component (Ab) containing a catalyst; and

a second purification step of continuously introducing the side cut component (As) to a diaryl carbonate purifying column B having a side cut discharge port, and continuously separating by distillation the component into three components, a column top component (Bt), a side cut component (Bs), and a column bottom component (Bb), thereby obtaining high-purity diaryl carbonate as the side cut component (Bs) (hereinafter, the first and second purification steps are also collectively referred to as a "separation and purification step").

The mixture containing diaryl carbonate is composed mainly of diaryl carbonate and may also contain, for example, unreacted alkyl aryl carbonate, a small amount of unreacted raw materials, a small amount of high-boiling by-products, and a catalytic component. Thus, the separation and purification step is carried out from the viewpoint of obtaining high-purity diphenyl carbonate from the mixture containing diaryl carbonate. In the separation and purification step, two distillation columns (the purifying column A having a side cut discharge port and the diaryl carbonate purifying column B having a side cut discharge port) are used. In the purifying column A, the mixture is continuously separated into, for example, a column top component (At) composed mainly of unreacted alkylaryl carbonate, a small amount of unreacted raw materials, and diaryl carbonate, a side cut component (As) containing diaryl carbonate, and a column bottom component (Ab) composed mainly of a small amount of high-boiling by-products, etc. and/or a catalytic component. The side cut component (As) of the purifying column can be continuously supplied to the diaryl carbonate purifying column B, and in this diaryl carbonate purifying column B, continuously separated into three components, a column top component (Bt), a side cut component (Bs), and a column bottom component (Bb), thereby obtaining high-purity diaryl carbonate as the side cut component (Bs). The side cut discharge port is installed in the purifying column A, whereby a large proportion of alkyl aryl carbonate can be separated by the purifying column A. This can suppress the alteration of alkyl aryl carbonate or diaryl carbonate while decreasing a load on the diaryl carbonate purifying column B. As a result, the amount of loss of diaryl carbonate ascribable to distillation can presumably be reduced. Such reduction in the amount of loss of diaryl carbonate can decrease alteration product waste, can further reduce an energy usage based on a production amount, and is presumably capable of reducing an environmental load in the production of high-purity diaryl carbonate.

In the separation and purification step, the purifying column A is preferably, for example, a continuous multistage distillation column that has a cylindrical body with a length $L_A$ (cm) and an inside diameter $D_A$ (cm), has a structure having an internal in the inside, and has an introduction port $A_1$ in an intermediate portion of the column and a side cut discharge port $A_2$ between the introduction port $A_1$ and the column bottom, wherein the number of stages of the internal from the introduction port $A_1$ through an upper portion is defined as $n_{A1}$, the number of stages of the internal between the introduction port $A_1$ and the side cut discharge port $A_2$ is defined as $n_{A2}$, the number of stages of the internal from the side cut discharge port $A_2$ through a lower part is defined as $n_{A3}$, and the total number of stages ($n_{A1} + n_{A2} + n_{A3}$) is defined as $n_A$, wherein $L_A$, $D_A$, $n_{A1}$, $n_{A2}$, $n_{A3}$, and $n_A$ respectively satisfy the following expressions (19) to (24):

$$800 \leq L_A \leq 3000 \text{ ... Expression (19)}$$

$$100 \leq D_A \leq 1000 \text{ ... Expression (20)}$$

$$1 \leq n_{A1} \leq 5 \text{ ... Expression (21)}$$

$$10 \leq n_{A2} \leq 19 \text{ ... Expression (22)}$$

$$10 \leq n_A \leq 50 \text{ ... Expression (24)}$$

In the method for industrially producing high-purity diaryl carbonate according to the present embodiment, particularly, the theoretical number of stages An from the column bottom to the side cut discharge port of the purifying column A, i.e., $n_{A2}$, is preferably in the range of 10 or more and 19 or less, more preferably in the range of 11 or more and 18 or less, further preferably in the range of 12 or more and 17 or less. When the theoretical number of stages An from the column bottom to the side cut discharge port of the purifying column A is equal to or more than the lower limit value described above, an XA concentration in the finally obtained diphenyl carbonate tends to be able to be lowered. When the theoretical number of stages is equal to or more than the lower limit value described above, the amount of loss of diaryl carbonate ascribable to distillation tends to be able to be reduced.

The diaryl carbonate purifying column B is preferably, for example, a continuous multistage distillation column that has a cylindrical body with a length $L_B$ (cm) and an inside diameter $D_B$ (cm), has a structure having an internal in the inside, and has an introduction port $B_1$ in an intermediate portion of the column and a side cut discharge port $B_2$ between the introduction port $B_1$ and the column bottom, wherein the number of stages of the internal from the introduction port $B_1$ through an upper portion is defined as $n_{B1}$, the number of stages of the internal between the introduction port $B_1$ and the side cut discharge port $B_2$ is defined as $n_{B2}$, the number of stages of the internal from the side cut discharge port $B_2$ through a lower portion is defined as $n_{B3}$, and the total number of stages ($n_{B1}$ + $n_{B2}$ + $n_{B3}$) is defined as $n_B$, wherein $L_B$, $D_B$, $n_{B1}$, $n_{B2}$, $n_{B3}$, and $n_B$ respectively satisfy the following expressions (25) to (30) :

$$1000 \leq L_B \leq 5000 \text{ ... Expression (25)}$$

$$100 \leq D_B \leq 1000 \text{ ... Expression (26)}$$

$$5 \leq n_{B1} \leq 20 \text{ ... Expression (27)}$$

$$12 \leq n_{B2} \leq 40 \text{ ... Expression (28)}$$

$$3 \leq n_{B3} \leq 15 \text{ ... Expression (29)}$$

$$20 \leq n_B \leq 70 \text{ ... Expression (30)}$$

All of these conditions are simultaneously satisfied, whereby high-purity diaryl carbonate tends to be able to be stably produced from the mixture containing diaryl carbonate at an industrial scale of 0.5 tons or more per hour for a long period of, for example, 2000 hours or longer, preferably 3000 hours or longer, further preferably 5000 hours or longer. The reason is not clear why the method of the present embodiment allows for the production of high-purity diaryl carbonate at an industrial scale having such excellent effects. The effects are presumably brought about by a combination of the purifying column A and the diaryl carbonate purifying column B which satisfy the conditions of the expressions (19) to (30). Preferred ranges of the respective factors will be given below.

When $L_A$ (cm) is 800 or more, separation efficiency tends to be improved because the internal that can be

installed in the inside of the purifying column A has a height sufficient therefor. For reducing the cost of equipment while achieving the desired separation efficiency, $L_A$ is preferably 3000 or less. $L_A$ (cm) is more preferably in the range of $1000 \le L_A \le 2500$, further preferably $1200 \le L_A \le 2000$.

When $D_A$ (cm) is 100 or more, the desired production amount can be achieved. For reducing the cost of equipment while achieving the desired production amount, $D_A$ is preferably 1000 or less. $D_A$ (cm) is more preferably in the range of $200 \le D_A \le 600$, further preferably $250 \le D_A \le 450$.

When $n_A$ is 20 or more, the desired high purity can be achieved because separation efficiency is improved. For reducing the cost of equipment while achieving the desired separation efficiency, $n_A$ is preferably 100 or less. When $n_A$ is 100 or less, long-term stable operation of the purifying column A is easy to perform because difference in pressure between the upper and lower portions of the column can be prevented from becoming too large. Besides, side reaction tends to be suppressed because a temperature in the lower portion of the column can be decreased. $n_A$ is more preferably in the range of $30 \le n_A \le 70$, further preferably $35 \le n_A \le 60$.

When $L_B$ (cm) is 1000 or more, separation efficiency tends to be improved because the internal that can be installed in the inside of the diaryl carbonate purifying column B has a height sufficient therefor. For reducing the cost of equipment while achieving the desired separation efficiency, $L_B$ is preferably 5000 or less. $L_B$ (cm) is more preferably in the range of $1500 \le L_B \le 3000$, further preferably $1700 \le L_B \le 2500$.

When $D_B$ (cm) is 100 or more, the desired production amount can be achieved. For reducing the cost of equipment while achieving the desired production amount, $D_B$ is preferably 1000 or less. $D_B$ (cm) is more preferably in the range of $150 \le D_B \le 500$, further preferably $200 \le D_B \le 400$.

When $n_B$ is 20 or more, the desired high purity can be achieved because separation efficiency as the whole column is improved. For reducing the cost of equipment while achieving the desired separation efficiency, $n_B$ is preferably 70 or less. When $n_B$ is 70 or less, long-term stable operation of the diaryl carbonate purifying column B is easy to perform because difference in pressure between the upper and lower portions of the column can be prevented from becoming large. Besides, side reaction tends to be suppressed because a temperature in the lower portion of the column can be decreased. $n_B$ is more preferably in the range of $25 \le n_B \le 55$, further preferably $30 \le n_B \le 50$. For stably obtaining the desired high-purity diaryl carbonate for a long time, $n_{B1}$, $n_{B2}$, and $n_{B3}$ are preferably in the range of $5 \le n_{B1} \le 20$, $12 \le n_{B2} \le 40$, and $3 \le n_{B3} \le 15$, respectively, more preferably in the range of $7 \le n_{B1} \le 15$, $12 \le n_{B2} \le 30$, and $3 \le n_{B3} \le 10$, respectively.

In the method for industrially producing high-purity diaryl carbonate according to the present embodiment, the ratio (SA/BsF) of a column cross section (SA ($m^2$)) of the purifying column A to a production amount (BsF (ton/hr)) as the discharged side cut component (Bs) is preferably in the range of 0.5 or more and 5 or less, more preferably in the range of 0.7 or more and 4.5 or less, further preferably in the range of 1 or more and 4 or less. When SA/BsF is equal to or more than the lower limit value described above, elevation in column bottom temperature ascribable to pressure loss can be suppressed and loss tends to be reduced. When the ratio is equal to or less than the upper limit value described above, the formation of by-products ascribable to elevation in residence time can be suppressed and loss tends to be reduced.

In the method for industrially producing high-purity diaryl carbonate according to the present embodiment, the purifying column A preferably comprises at least one reboiler selected from the group consisting of forced circulation type, cross pipe falling film type, and thin-film evaporation type, more preferably comprises at least one reboiler selected from the group consisting of forced circulation type and cross pipe falling film type, and particularly preferably comprises a reboiler of forced circulation type. When the purifying column A comprises such a reboiler, the tube wall temperature of the reboiler can be decreased and high-boiling material formation ascribable to local overheat tends to be able to be prevented.

In carrying out the present embodiment, the purifying column A is preferably operated at a column bottom temperature ($T_A$) of 185 to 280°C and a column top pressure ($P_A$) of 1000 to 20000 Pa, and the diaryl carbonate purifying column B is preferably operated at a column bottom temperature ($T_B$) of 185 to 280°C and a column top pressure ($P_B$) of 1000 to 20000 Pa.

When $T_A$ is 185°C or higher, equipment that retains high vacuum is not necessary because the column top pressure does not have to be lower. Also, equipment can be downsized. When $T_A$ is 280°C or lower, the formation of high-boiling by-products during distillation tends to be able to be suppressed. $T_A$ is in the range of more preferably 190 to 240°C, further preferably 195 to 230°C.

When $P_A$ is 1000 Pa or higher, large equipment is not necessary for retaining high vacuum. When $P_A$ is 20000 Pa or lower, by-products tend to be able to be suppressed because of a low distillation temperature. $P_A$ is in the range of more preferably 2000 to 15000 Pa, further preferably 3000 to 13000 Pa.

When $T_B$ is 185°C or higher, equipment that retains high vacuum is not necessary because the column top pressure does not have to be lower. Also, equipment can be downsized. When $T_B$ is 280°C or lower, the formation of high-boiling by-products during distillation tends to be able to be suppressed. $T_B$ is in the range of more preferably 190 to 240°C, further preferably 195 to 230°C.

When $P_B$ is 1000 Pa or higher, large equipment is not necessary for retaining high vacuum. When $P_B$ is 20000 Pa or lower, by-products tend to be able to be suppressed because of a low distillation temperature. $P_B$ is in the range of more preferably 2000 to 15000 Pa, further preferably 3000 to 13000 Pa.

The purifying column A and the diaryl carbonate purifying column B may have the same inside diameter from the respective upper to lower portions of the columns or may have partially different inside diameters as long as $D_A$ and $D_B$ fall within the ranges described above. In these continuous multistage distillation columns, for example, the upper inside diameters of the columns may be smaller or larger than the lower inside diameters of the columns.

In the method for industrially producing high-purity diaryl carbonate according to the present embodiment, the ratio ($D_{B1}/D_{B2}$) of an upper column diameter ($D_{B1}$ (m)) to a lower column diameter ($D_{B2}$ (m)) in the purifying column B is preferably in the range of 0.1 or more and 0.9 or less, more preferably in the range of 0.2 or more and 0.9 or less, further preferably in the range of 0.3 or more and 0.8 or less. When $D_{B1}/D_{B2}$ is equal to or more than the lower limit value described above, elevation in column bottom temperature ascribable to elevation in pressure loss can be suppressed and loss tends to be reduced. When the ratio is equal to or less than the upper limit value described above, by-product formation ascribable to elevation in residence time can be suppressed and loss tends to be reduced.

Each of the purifying column A and the diaryl carbonate purifying column B for use in the separation and purification step is preferably a distillation column having a tray and/or a packing as the internal. The internal described in the present embodiment means a portion where the contact between a gas and a liquid is actually performed in each distillation column. Such a tray is preferably, for example, a bubble-cap tray, a sieve tray, a valve tray, a countercurrent tray, a Superfrac tray, or a Max-Frac tray, and the packing is preferably a random packing such as a Raschig ring, a Lessing ring, a pall ring, a Berl saddle, an Intalox saddle, Dixon Packing, Mc MAHON Packing, or Heli Pack, or a structured packing such as Mellapak, Gempak, Techno Pack, Flexipac, Sulzer Packings, Goodloe Packing, or Glitsch Grid. The "number of stages of the internal" means the number of trays in the case of a tray and means the theoretical number of stages in the case of a packing. Thus, in the case of a multistage distillation column having both of a tray portion and a portion filled with a packing, the number of stages is the total sum of the number of trays and the theoretical number of stages.

The purifying column A in the separation and purification step preferably has a packing as the internal and preferably has a structured packing as the packing. The diaryl carbonate purifying column B preferably has a packing as the internal and preferably has one or two or more structured packings.

In the method for industrially producing high-purity diaryl carbonate according to the present embodiment, the packing of the purifying column A and/or the purifying column B is a structured packing, and the structured packing preferably comprises at least one selected from the group consisting of Mellapak, Gempak, Techno Pack, Flexipac, Sulzer Packings, Goodloe Packing, Glitsch Grid, and a gauze packing, more preferably comprises at least one selected from the group consisting of Mellapak, Flexipac, Sulzer Packings, and a gauze packing, and is particularly preferably Mellapak. When the purifying column A contains such a structured packing, pressure loss is reduced and a production amount range that permits operation tends to be elevated.

The mixture to be introduced to the purifying column A may contain other components in addition to diaryl carbonate. For example, when the mixture is a reaction mixture obtained through at least one reaction selected from the group consisting of (i) transesterification reaction of dialkyl carbonate and aryl alcohol, (ii) disproportionation reaction of alkyl aryl carbonate, and (iii) transesterification reaction of alkyl aryl carbonate and aryl alcohol, in the presence of a catalyst, the reaction mixture usually contains the catalyst, dialkyl carbonate, an aromatic monohydroxy compound, alkyl aryl carbonate, by-products, and the like in addition to diaryl carbonate. The by-products include by-products having a relatively low boiling point, such as alkyl aryl ether, Fries rearrangement products of alkyl aryl carbonate or diaryl carbonate or their derivatives, modification products of diaryl carbonate, and high-boiling by-products such as structurally unknown high-boiling substances. For example, in the case of producing diphenyl carbonate with dimethyl carbonate and phenol as raw materials, anisole, methyl salicylate, phenyl salicylate, xanthone, phenyl methoxybenzoate, 1-phenoxycarbonyl-2-phenoxycarboxy-phenylene, and the like are present as reaction by-products, and a small amount of structurally unknown high-boiling by-products obtained through a further reaction is usually contained. Specifically, 0.05 to 2% by mass of dialkyl carbonate, 1 to 20% by mass of phenol, 0.05 to 2% by mass of alkyl phenyl ether, 10 to 45% by mass of alkyl phenyl carbonate, 50 to 80% by mass of diphenyl carbonate, 0.1 to 5% by mass of high-boiling by-products, and 0.001 to 5% by mass of the catalyst are contained. Therefore, it is preferred to continuously supply this continuously discharged column bottom liquid directly to the purifying column A in the separation and purification step.

The composition of the reaction mixture varies depending on conditions of the transesterification reaction of dialkyl carbonate and aryl alcohol, the type and amount of the catalyst, etc. A reaction mixture having almost constant composition can be produced as long as the transesterification reaction is performed under constant conditions. Therefore, the composition of the reaction mixture to be supplied to the purifying column A is almost constant. However, in the separation and purification step, the reaction mixture can be separated with almost the same separation efficiency even if its composition varies within the range described above. This is one of the features of the separation and purification step used in the present embodiment.

In the separation and purification step, for example, a column bottom liquid of a second continuous multistage distillation column for transesterification reaction of dialkyl carbonate and aryl alcohol, when continuously supplied into the purifying column A, may be supplied in a liquid form from an introduction port installed at one or several locations below the intermediate portion of the purifying column A, or supply into the purifying column A through a reboiler from a piping disposed in the lower portion of the reboiler of the purifying column A is also a preferred method. The amount of the column bottom liquid of the second continuous multistage distillation column supplied into the purifying column A varies depending on the production amount of the high-purity diaryl carbonate to be produced, the concentration of the diaryl carbonate in the reaction mixture, separation conditions for the purifying column A, etc., and is usually approximately 2 ton/hr or more, preferably approximately 6 ton/hr or more, further preferably approximately 10 ton/hr or more.

The mixture continuously supplied into the purifying column A is separated into, for example, a column top component (At) having a large proportion of compounds having a lower boiling point than that of diaryl carbonate, such as unreacted raw materials, alkyl aryl ether, and alkyl aryl carbonate, a side cut component (As) containing diaryl carbonate, and a column bottom component (Ab) containing a small amount of diaryl carbonate, the catalyst, and high-boiling by-products. The column bottom component (Ab) may contain a small amount of alkyl aryl carbonate. These organic materials in the column bottom component help to dissolve a catalytic component and maintain a liquid form. Usually, the whole amount or a portion of this column bottom component (Ab) is continuously suppled as a catalytic component for transesterification reaction directly into a first continuous multistage distillation column and/or a second continuous multistage distillation column for transesterification reaction of dialkyl carbonate and aryl alcohol, and circulated and reused. In some cases, this component is separated from organic materials in a catalyst recovery step, then regenerated as a catalyst, and circulated and reused.

In the separation and purification step, for example, by-products having a higher boiling point than that of diphenyl carbonate, such as phenyl salicylate, xanthone, phenyl methoxybenzoate, and 1-phenoxycarbonyl-2-phenoxycarboxy-phenylene, and a catalytic component are almost completely separated as the column bottom component (Ab) in this purifying column A. The side cut component (As) which can be discharged with these high-boiling by-products rarely contained therein is also one of the features of the separation and purification step. In the separation and purification step, 80% or more, preferably 84% or more, more preferably 87% or more, of diaryl carbonate in the mixture continuously supplied into the purifying column A can be discharged from a side cut. In the separation and purification step, usually 10 to 40% by mass of the continuously supplied liquid is continuously discharged as the column top component (At) from the column top, 40 to 70% by mass thereof is continuously discharged as the side cut component (As) from the side cut, and 10 to 40% by mass thereof is continuously discharged as the column bottom component (Ab) from the column bottom, though depending on the composition of the mixture supplied to the purifying column A. The composition of the side cut component (As) comprises 0 to 10% by mass of dialkyl carbonate, 0 to 10% by mass of aryl alcohol, 0 to 10% by mass of alkyl aryl ether, 1 to 20% by mass of alkyl aryl carbonate, and 50 to 99% by mass of diaryl carbonate, and the content of high-boiling by-products is usually 8000 ppm or less, preferably 5000 ppm or less, more preferably 3000 ppm or less.

In the separation and purification step, the reflux ratio of the purifying column A is preferably in the range of 1 to 15, more preferably 2 to 12, further preferably 2.5 to 6.5.

The amount of the side cut component (As) continuously discharged from the column top of the purifying column A is usually 10 to 40% by mass of the mixture supplied into the purifying column A, as described above. This amount is continuously supplied directly into the purifying column B from the introduction port $B_1$ disposed in the intermediate portion of the diaryl carbonate purifying column B, and continuously separated into three components, a column top component (Bt), a side cut component (Bs), and a column bottom component (Bb). All of components having a lower boiling point than that of diaryl carbonate contained in the side cut component (As) of the purifying column A supplied into the purifying column B are continuously discharged as the column top component (Bt) from the column top, while a small amount of a liquid is continuously discharged from the column bottom. The column top component (Bt) contains a small amount of diaryl carbonate, the amount of which is usually 0.01 to 1.0% by mass, preferably 0.1 to 0.8% by mass, based on the supplied diaryl carbonate. The diaryl carbonate in this column top component (Bt) is separated by another distillation column for separating the column top component (Bt) and recovered. Such separation of the diaryl carbonate as a column bottom component of another distillation column and its recovery by return into the purifying column A and/or the diaryl carbonate purifying column B is also a preferred method.

The column bottom component (Bb) contains diaryl carbonate and a small amount of high-boiling by-products concentrated into approximately several %. A very small amount of diaryl carbonate in the column bottom component (Bb) discharged from the column bottom is also one of the features of the present embodiment. The amount is usually 1 to 15% by mass based on the supplied diaryl carbonate.

Highly pure diaryl carbonate is continuously discharged at a flow rate of 0.5 ton/hr or more, preferably 1 ton/hr or more, more preferably 3 ton/hr or more, further preferably 5 ton/hr or more, from the side cut discharge port $B_2$. This amount usually corresponds to approximately 70 to 90% by mass of the diaryl carbonate supplied into the purifying column B.

The purity of the diaryl carbonate obtained as the side cut component (Bs) in the separation and purification step is usually 99% or more, preferably 99.9% or more, more preferably 99.99% or more, further preferably 99.999% or more. In the present embodiment, the high purity means 99% or more and is preferably 99.9% or more, more preferably 99.99% or more, further preferably 99.999% or more. The contents of high-boiling impurities in high-purity diphenyl carbonate obtained when the transesterification reaction step of dialkyl carbonate and aryl alcohol and the separation and purification step are carried out with dimethyl carbonate and phenol as raw materials are preferably 30 ppm or less, more preferably 10 ppm or less, further preferably 1 ppm or less, of phenyl salicylate, preferably 30 ppm or less, more preferably 10 ppm or less, further preferably 1 ppm or less, of xanthone, preferably 30 ppm or less, more preferably 10 ppm or less, further preferably 1 ppm or less, of phenyl methoxybenzoate, and preferably 30 ppm or less, more preferably 10 ppm or less, further preferably 5 ppm or less, of 1-phenoxycarbonyl-2-phenoxycarboxy-phenylene. The total content of these high-boiling by-products is preferably 100 ppm or less, more preferably 50 ppm or less, further preferably 10 ppm or less.

The content of the diaryl carbonate in the side cut component (As) is preferably 80% by mass or more, and the content of the diaryl carbonate in the side cut component (Bs) is preferably 99.1% by mass or more. In this context, the upper limit value of the content of the diaryl carbonate in the side cut component (Bs) is not particularly limited and is, for example, 99.8% by mass.

The content of the diaryl carbonate in the side cut component (As) is preferably 80% by mass or more, more preferably 85% by mass or more. The upper limit of the content of the diaryl carbonate in the side cut component (As) is not particularly limited and may be, for example, 99.5% or less or may be 99.2% or less.

In the present embodiment, the purity of the diaryl carbonate (e.g., diphenyl carbonate) and the content of impurities can be measured by methods described in Examples mentioned later.

In the present embodiment, in the case of using halogen-free raw materials and a catalyst, the halogen content of the resulting diaryl carbonate is preferably 0.1 ppm or less, more preferably 10 ppb or less, further preferably 1 ppb or less (which falls outside a detection limit in ion chromatography).

In the separation and purification step, the reflux ratio of the diaryl carbonate purifying column B is in the range of 0.01 to 10, preferably in the range of 0.1 to 8, further preferably 0.5 to 5.

The material constituting the purifying column A, the diaryl carbonate purifying column B, and a wetted portion used in the present embodiment is mainly a metal material such as carbon steel or stainless steel, and stainless steel is preferred from the viewpoint of the quality of the produced diaryl carbonate.

By carrying out the method of the present embodiment, for example, high-purity diaryl carbonate necessary for producing high-quality and high-performance aromatic polycarbonate can be produced at an industrial scale of 0.5 tons or more, preferably 1 ton or more, more preferably 2 tons or more, further preferably 3 tons or more, per hour from cyclic carbonate and aryl alcohol (e.g., phenol). In the present embodiment, the industrial scale means 0.5 tons or more per hour and is preferably 1 ton or more, more preferably 2 tons or more, further preferably 3 tons or more.

Hereinafter, a method for producing the mixture containing diaryl carbonate will be described in detail.

Examples of the method for producing a reaction mixture containing alkyl aryl carbonate through (i) transesterification reaction of dialkyl carbonate and aryl alcohol (hereinafter, also referred to as an "aromatic monohydroxy compound") include, but are not particularly limited to, a method of performing transesterification reaction of dialkyl carbonate and an aromatic monohydroxy compound in the presence of a homogeneous catalyst, continuously discharging a reaction mixture containing an alcohol from the column top, and continuously discharging a reaction mixture containing alkyl aryl carbonate from the column bottom.

It is preferred to continuously supply the reaction mixture containing alkyl aryl carbonate thus obtained into a reactive distillation column consisting of a continuous multistage distillation column containing a homogeneous catalyst, simultaneously perform transesterification reaction and distillation in the column, continuously discharge a low-boiling reaction mixture containing dialkyl carbonate thus formed in a gas form from an upper portion of the column, and continuously discharge a high-boiling reaction mixture containing diaryl carbonate in a liquid form from a lower portion of the column. This transesterification reaction includes a reaction of exchanging an alkoxy group of the alkyl aryl carbonate with an aryloxy group of the aromatic monohydroxy compound present in the system to eliminate alcohols, and a reaction of converting the alkyl aryl carbonate into diaryl carbonate and dialkyl carbonate through disproportionation reaction which is transesterification reaction between two molecules of the alkyl aryl carbonate. In the reactive distillation column, the disproportionation reaction of the alkyl aryl carbonate mainly takes place.

In this context, the reaction mixture containing alkyl aryl carbonate, which is used as a raw material, may have a high purity or may contain other compounds. The reaction mixture may contain, for example, the dialkyl carbonate and/or the aromatic monohydroxy compound used for obtaining this alkyl aryl carbonate, and may contain compounds and reaction by-products formed in this step and/or other steps, for example, alcohols, alkyl aryl ethers, and diaryl carbonate. Direct use of the transesterification reaction mixture of the dialkyl carbonate and the aromatic monohydroxy compound as a raw material without separation of an unreacted substance or the catalyst is also a preferred method. In the case of carrying out an industrial process as in the present embodiment, the dialkyl carbonate and the aromatic

monohydroxy compound for use in obtaining the raw material alkyl aryl carbonate are preferably dialkyl carbonate and an aromatic monohydroxy compound that are newly introduced into the reaction system as well as those recovered in this step and/or other steps.

The continuous multistage distillation column for use as the reactive distillation column is preferably a distillation column having a tray and/or a packing as the internal. The internal described in the present embodiment means a portion where the contact between a gas and a liquid is actually performed in the distillation column. Such a tray is preferably, for example, a bubble-cap tray, a sieve tray, a valve tray, a countercurrent tray, a Superfrac tray, or a Max-Frac tray, and the packing is preferably a random packing such as a Raschig ring, a Lessing ring, a pall ring, a Berl saddle, an Intalox saddle, Dixon Packing, Mc MAHON Packing, or Heli Pack, or a structured packing such as Mellapak, Gempak, Techno Pack, Flexipac, Sulzer Packings, Goodloe Packing, or Glitsch Grid.

In this context, the reaction of the alkyl aryl carbonate with the aromatic monohydroxy compound present in the system has a very small equilibrium constant and furthermore, has a slow reaction rate, and the disproportionation reaction, a main reaction, of the alkyl aryl carbonate is also an equilibrium reaction having a small equilibrium constant and a slow reaction rate. The continuous multistage distillation column for reactive distillation where such a reaction is performed is preferably a multistage distillation column having both a packing and a tray in the internal. In this distillation column, a portion filled with the packing is preferably installed in an upper portion, and the tray portion is preferably installed in a lower portion. The packing is preferably a structured packing, and one or two or more structured packings are preferably used. The structured packing is preferably at least one selected from Mellapak, Gempak, Techno Pack, Flexipac, Sulzer Packings, Goodloe Packing, and Glitsch Grid.

For the reactive distillation column, the tray of the internal is preferably a sieve tray having a sieve portion and a downcomer portion because of an excellent relationship between functions and the cost of equipment. The sieve tray preferably has 100 to 1000 holes per $m^2$ of the area of the sieve portion. The number of holes is more preferably 120 to 900, further preferably 150 to 800, per $m^2$ of the area. The cross section per hole of the sieve tray is preferably 0.5 to 5 $cm^2$. The cross section per hole is more preferably 0.7 to 4 $cm^2$, further preferably 0.9 to 3 $cm^2$. The case is particularly preferred where the sieve tray has 100 to 1000 holes per $m^2$ of the area of the sieve portion, and the cross section per hole is 0.5 to 5 $cm^2$. A continuous multistage distillation column is particularly preferred in which the structured packing is at least one selected from Mellapak, Gempak, Techno Pack, Flexipac, Sulzer Packings, Goodloe Packing, and Glitsch Grid, the sieve tray has 100 to 1000 holes per $m^2$ of the area of the sieve portion, and the cross section per hole of the sieve tray is 0.5 to 5 $cm^2$.

In the present embodiment, in the case of carrying out a reactive distillation step, it is preferred to continuously supply raw materials containing alkyl aryl carbonate into a continuous multistage distillation column containing a homogeneous catalyst, simultaneously perform a reaction and distillation in the column, continuously discharge a low-boiling reaction mixture containing dialkyl carbonate and alcohols thus formed in a gas form from an upper portion of the column, and continuously discharge a high-boiling reaction mixture containing diaryl carbonate as a main reaction product in a liquid form from a lower portion of the column to continuously produce diaryl carbonate. In the present embodiment, the method for allowing the reactive distillation column to contain a catalyst can be any method. The homogeneous catalyst is soluble in raw materials or a reaction liquid and is therefore preferably supplied into the distillation column from a position above the intermediate portion of the distillation column. In this case, a solution of the catalyst dissolved in raw materials or a reaction liquid may be introduced together with the raw materials, or this catalyst solution may be introduced from an introduction port different from that for the raw materials. The catalyst that is used for producing raw materials containing alkyl aryl carbonate and contained in the raw materials may be used directly as a catalyst in this reactive distillation step, and this is also a preferred method. If necessary, the catalyst described above may be newly added. The amount of the catalyst used here differs depending on difference in the type of the catalyst used, the types and quantitative ratio of the raw materials, and reaction conditions such as a reaction temperature and a reaction pressure. The catalyst is used at usually 0.0001 to 30% by mass, preferably 0.005 to 10% by mass, more preferably 0.001 to 1% by mass, in terms of a ratio to the total mass of the raw materials.

The low-boiling reaction mixture to be continuously discharged from an upper portion of the reactive distillation column may contain, for example, the aromatic monohydroxy compound present in the system, alkyl aryl ethers, and unreacted alkyl aryl carbonate. This low-boiling reaction mixture is preferably circulated and reused in a transesterification reactor of dialkyl carbonate and an aromatic monohydroxy compound. Reflux operation of condensing a gas component discharged from the column top of the reactive distillation column, and then returning a portion of the condensate to the upper portion of the distillation column is also a preferred method. In this case, the reflux ratio is in the range of 0.05 to 10, preferably 0.08 to 5, further preferably 0.1 to 2.

The raw materials containing alkyl aryl carbonate, when continuously supplied into the reactive distillation column, are preferably supplied in a liquid form and/or in a gas form from an introduction port installed at one or several locations below an upper gas discharge port of the distillation column and in an upper portion or an intermediate portion of the column. In the case of using a distillation column having a packing portion in an upper portion and a tray portion in a lower portion, at least one introduction port is preferably installed between the packing portion and the tray portion.

When the packing is formed from a plurality of (two or more) structured packings, an introduction port may be installed in space flanked by these structured packings, and this is also a preferred method.

The reaction time of the transesterification reaction presumably corresponds to an average residence time of a reaction liquid in the reactive distillation column. This differs depending on the shape and number of stages of the internal of the distillation column, the amount of raw materials supplied, the type and amount of a catalyst, reaction conditions, etc. and is usually 0.01 to 10 hours, preferably 0.05 to 5 hours, more preferably 0.1 to 3 hours.

The reaction temperature of the transesterification reaction differs depending on the type of the raw material compound used and the type and amount of a catalyst, and is usually 100 to 350°C. A higher reaction temperature is preferred for enhancing a reaction rate and however, is not preferred because such a high reaction temperature easily causes side reaction and increases by-products such as Fries rearrangement products of alkyl aryl ether or diaryl carbonate, or ester compounds thereof. In this respect, the reaction temperature is in the range of preferably 130 to 280°C, more preferably 150 to 260°C, further preferably 180 to 240°C. The reaction pressure differs depending on the type and composition of the raw material compound used, the reaction temperature, etc., and can be any of reduced pressure, normal pressure, and increased pressure. Usually, the reaction is performed at a column top pressure in the range of 0.1 to $2 \times 10^7$ Pa, preferably $10^3$ to $10^6$ Pa, more preferably $5 \times 10^3$ to $10^5$ Pa.

The high-boiling reaction mixture containing diaryl carbonate continuously discharged from a lower portion of the reactive distillation column usually contains the catalyst, dialkyl carbonate, an aromatic monohydroxy compound, alkyl aryl carbonate, by-products, and the like in addition to the diaryl carbonate. The by-products include by-products having a relatively low boiling point, such as alkyl aryl ether, Fries rearrangement products of alkyl aryl carbonate or diaryl carbonate or their derivatives, modification products of diaryl carbonate, and high-boiling by-products such as structurally unknown high-boiling substances. For example, in the case of producing diphenyl carbonate with dimethyl carbonate and phenol as raw materials, anisole, methyl salicylate, phenyl salicylate, xanthone, phenyl methoxybenzoate, 1-phenoxycarbonyl-2-phenoxycarboxy-phenylene, and the like are present as reaction by-products, and a small amount of structurally unknown high-boiling by-products obtained through a further reaction is usually contained.

The dialkyl carbonate for use in (i) transesterification reaction of dialkyl carbonate and aryl alcohol is represented by the general formula (I):

$$R^1OCOOR^1 \qquad (I)$$

In this formula, $R^1$ represents an alkyl group having 1 to 10 carbon atoms, an alicyclic group having 3 to 10 carbon atoms, or an aralkyl group having 6 to 10 carbon atoms. Examples of such $R^1$ include: alkyl groups such as methyl, ethyl, propyl (each isomer), allyl, butyl (each isomer), butenyl (each isomer), pentyl (each isomer), hexyl (each isomer), heptyl (each isomer), octyl (each isomer), nonyl (each isomer), decyl (each isomer), and cyclohexylmethyl; alicyclic groups such as cyclopropyl, cyclobutyl, cyclopentyl, cyclohexyl, and cycloheptyl; and aralkyl groups such as benzyl, phenethyl (each isomer), phenylpropyl (each isomer), phenylbutyl (each isomer), and methylbenzyl (each isomer). These alkyl groups, alicyclic groups, and aralkyl groups may be substituted by other substituents, for example, a lower alkyl group, a lower alkoxy group, a cyano group, and/or halogen and may have an unsaturated bond.

Examples of the dialkyl carbonate having such $R^1$ include dimethyl carbonate, diethyl carbonate, dipropyl carbonate (each isomer), diallyl carbonate, dibutenyl carbonate (each isomer), dibutyl carbonate (each isomer), dipentyl carbonate (each isomer), dihexyl carbonate (each isomer), diheptyl carbonate (each isomer), dioctyl carbonate (each isomer), dinonyl carbonate (each isomer), didecyl carbonate (each isomer), dicyclopentyl carbonate, dicyclohexyl carbonate, dicycloheptyl carbonate, dibenzyl carbonate, diphenethyl carbonate (each isomer), di(phenylpropyl) carbonate (each isomer), di(phenylbutyl) carbonate (each isomer), di(chlorobenzyl) carbonate (each isomer), di(methoxybenzyl) carbonate (each isomer), di(methoxymethyl) carbonate, di(methoxyethyl) carbonate (each isomer), di(chloroethyl) carbonate (each isomer), and di(cyanoethyl) carbonate (each isomer).

Among them, dialkyl carbonate wherein $R^1$ is an alkyl group having 4 or less carbon atoms and containing no halogen is preferably used, and dimethyl carbonate is particularly preferred. The preferred dialkyl carbonate is further preferably dialkyl carbonate produced in a state substantially free of halogen, and is produced from, for example, alkylene carbonate substantially free of halogen and an alcohol substantially free of halogen.

The aryl alcohol (aromatic monohydroxy compound) is represented by the following general formula (II) and can be any compound in which a hydroxyl group is bonded directly to an aromatic group:

$$Ar^1OH \qquad (II)$$

In the formula, $Ar^1$ represents an aromatic group having 5 to 30 carbon atoms. Examples of the aromatic monohydroxy compound having such $Ar^1$ include: phenol; various alkylphenols such as cresol (each isomer), xylenol (each isomer), trimethylphenol (each isomer), tetramethylphenol (each isomer), ethylphenol (each isomer), propylphenol (each isomer), butylphenol (each isomer), diethylphenol (each isomer), methylethylphenol (each isomer), methylpropylphenol (each isomer), dipropylphenol (each isomer), methylbutylphenol (each isomer), pentylphenol (each isomer), hexylphenol (each isomer), and cyclohexylphenol (each isomer); various alkoxyphenols such as methoxyphenol (each isomer) and ethoxyphenol (each isomer); arylalkylphenols such as phenylpropylphenol (each isomer); naphthol (each isomer) and various substituted naphthols; and heteroaromatic monohydroxy compounds such as hydroxypyridine (each isomer), hydroxycoumarin (each isomer), and hydroxyquinoline (each isomer). Among these aromatic monohydroxy compounds, unsubstituted or substituted phenol wherein $Ar^1$ consists of an aromatic group having 6 to 10 carbon atoms is preferably used, and unsubstituted phenol is particularly preferred. Among these aromatic monohydroxy compounds, a compound substantially free of halogen is preferably used.

The quantitative ratio between the dialkyl carbonate and the aromatic monohydroxy compound for use in obtaining the raw material reaction mixture containing alkyl aryl carbonate is 0.5 to 3 in terms of a molar ratio. When the quantitative ratio falls outside this range, unreacted materials remain in a large amount with respect to a necessary amount of the alkyl aryl carbonate of interest, which is not efficient. Also, a lot of energy is required for recovering these unreacted materials. In this respect, this molar ratio is more preferably 0.5 to 5, further preferably 1 to 3.

Examples of the catalyst used here include, but are not particularly limited to, homogeneous catalysts that contain a metal such as Pb, Cu, Zn, Fe, Co, Ni, Al, Ti, V, or Sn and are soluble in the reaction system. Thus, a catalyst species in which such a metal component is bonded to an organic group is preferably used. As a matter of course, these catalytic components may be reacted with organic compounds present in the reaction system, for example, aliphatic alcohols, aromatic monohydroxy compounds, alkyl aryl carbonates, diaryl carbonates, or dialkyl carbonates, or may be heat-treated with raw materials or products prior to the reaction. The catalyst used here preferably has a high solubility in a reaction liquid under reaction conditions. In this respect, preferred examples of the catalyst include, but are not particularly limited to: PbO, $Pb(OH)_2$, and $Pb(OPh)_2$; $TiCl_4$, $Ti(OMe)_4$, $(MeO)Ti(OPh)_3$, $(MeO)_2Ti(OPh)_2$, $(MeO)_3Ti(OPh)$, and $Ti(OPh)_4$; $SnCl_4$, $Sn(OPh)_4$, $Bu_2SnO$, and $Bu_2Sn(OPh)_2$; $FeCl_3$, $Fe(OH)_3$, and $Fe(OPh)_3$; and these catalysts treated with phenol or a reaction liquid.

In the present embodiment, it is particularly preferred to use halogen-free raw materials and a catalyst. In this case, the produced diaryl carbonate is totally free of halogen and is therefore important as a raw material in industrially producing polycarbonate by a transesterification method. This is because the presence of halogen even in an amount smaller than 1 ppm, for example, in polymerization raw materials inhibits polymerization reaction, reduces the physical properties of the formed polycarbonate, and causes stain.

In the method for industrially producing high-purity diaryl carbonate according to the present embodiment, other examples of the method for obtaining the mixture to be introduced to the purifying column A include, but are not particularly limited to, a method using two continuous multistage distillation columns as described below.

Specifically, the method for industrially producing high-purity diaryl carbonate according to the present embodiment preferably further comprises the steps of:

continuously supplying dialkyl carbonate and aryl alcohol into a first continuous multistage distillation column containing a catalyst, simultaneously performing a reaction and distillation in the first continuous multistage distillation column, continuously discharging a first column low-boiling reaction mixture containing alcohols thus formed in a gas form from an upper portion of the first continuous multistage distillation column, and continuously discharging a first column high-boiling reaction mixture containing alkyl aryl carbonate thus formed in a liquid form from a lower portion of the first continuous multistage distillation column to continuously produce alkyl aryl carbonate; and

continuously supplying the first column high-boiling reaction mixture into a second continuous multistage distillation column containing a catalyst, simultaneously performing a reaction and distillation in the second continuous multistage distillation column, continuously discharging a second column low-boiling reaction mixture containing dialkyl carbonates thus formed in a gas form from an upper portion of the second continuous multistage distillation column, and continuously discharging a second column high-boiling reaction mixture containing diaryl carbonate thus formed in a liquid form from a lower portion of the second continuous multistage distillation column to continuously produce diaryl carbonate (hereinafter, these two steps are also collectively referred to as a "step (II)").

The second column high-boiling reaction mixture obtained in the step (II) is preferably used as the mixture to be introduced to the purifying column A.

In the method for industrially producing high-purity diaryl carbonate according to the present embodiment, the diaryl carbonate is preferably diphenyl carbonate.

Examples of the method for obtaining the raw material dialkyl carbonate for use in the step (II) include, but are not particularly limited to, a method of performing a step (I) of continuously producing dialkyl carbonate and diols at an industrial scale from cyclic carbonate and aliphatic monovalent alcohols. The reaction of the step (I) is reversible transesterification reaction represented by the following formula:

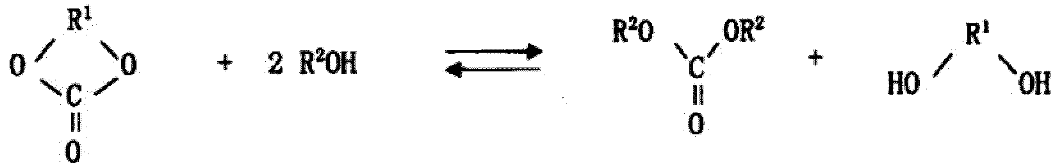

wherein $R^1$ represents a divalent group $-(CH_2)m-$ (m is an integer of 2 to 6), one or more hydrogen atoms of which are optionally replaced with an alkyl group having 1 to 10 carbon atoms or an aryl group; and $R^2$ represents a monovalent aliphatic group having 1 to 12 carbon atoms, one or more hydrogen atoms of which are optionally replaced with an alkyl group having 1 to 10 carbon atoms or an aryl group.

Examples of such cyclic carbonate that is preferably used include alkylene carbonates such as ethylene carbonate and propylene carbonate, 1,3-dioxacyclohex-2-one, and 1,3-dioxacyclohept-2-one. Ethylene carbonate and propylene carbonate are further preferably used from the viewpoint of easy availability, etc., and ethylene carbonate is particularly preferably used.

The aliphatic monovalent alcohols used have a lower boiling point than that of diols to be formed. Thus, examples of the aliphatic monovalent alcohols, which may vary depending on the type of the cyclic carbonate used, include methanol, ethanol, propanol (each isomer), allyl alcohol, butanol (each isomer), 3-buten-1-ol, amyl alcohol (each isomer), hexyl alcohol (each isomer), heptyl alcohol (each isomer), octyl alcohol (each isomer), nonyl alcohol (each isomer), decyl alcohol (each isomer), undecyl alcohol (each isomer), dodecyl alcohol (each isomer), cyclopentanol, cyclohexanol, cycloheptanol, cyclooctanol, methylcyclopentanol (each isomer), ethylcyclopentanol (each isomer), methylcyclohexanol (each isomer), ethylcyclohexanol (each isomer), dimethylcyclohexanol (each isomer), diethylcyclohexanol (each isomer), phenylcyclohexanol (each isomer), benzyl alcohol, phenethyl alcohol (each isomer), and phenylpropanol (each isomer). These aliphatic monovalent alcohols may be substituted by substituents such as halogen, a lower alkoxy group, a cyano group, an alkoxycarbonyl group, an aryloxycarbonyl group, an acyloxy group, or a nitro group.

Among such aliphatic monovalent alcohols, alcohols having 1 to 6 carbon atoms are preferably used, and alcohols having 1 to 4 carbon atoms, i.e., methanol, ethanol, propanol (each isomer), and butanol (each isomer), are further preferred. In the case of using ethylene carbonate or propylene carbonate as cyclic carbonate, methanol and ethanol are preferred, and methanol is particularly preferred.

The method for allowing the reactive distillation column to contain a catalyst in performing reactive distillation in the step (I) can be any method. In the case of, for example, a homogeneous catalyst soluble in a reaction liquid under reaction conditions, a liquid phase in the reactive distillation column can be allowed to contain the catalyst by continuously supplying the catalyst into the reactive distillation column. Alternatively, in the case of an inhomogeneous catalyst insoluble in a reaction liquid under reaction conditions, the reaction system can be allowed to contain the catalyst by placing a solid catalyst in the reactive distillation column. These methods may be used in combination.

The homogeneous catalyst, when continuously supplied into the reactive distillation column, may be supplied simultaneously with the cyclic carbonate and/or the aliphatic monovalent alcohol or may be supplied to a position different from that for raw materials. A region where the reaction actually progresses in the distillation column is located below a catalyst supply position. It is therefore preferred to supply the catalyst to a region between the column top and a portion slightly lower than a raw material supply position. Plates containing the catalyst are preferably five or more stages, more preferably seven or more stages, further preferably ten or more stages.

In the case of using an inhomogeneous solid catalyst, the number of stages containing the catalyst is preferably 5 or more, more preferably 7 or more, further preferably 10 or more. A solid catalyst also having effects as a distillation column packing may be used.

Examples of the catalyst for use in the step (I) include, but are not particularly limited to:

alkali metals and alkaline earth metals such as lithium, sodium, potassium, rubidium, cesium, magnesium, calcium, strontium, and barium;
basic compounds such as hydride, hydroxide, alkoxides, aryloxides, and amides of alkali metals and alkaline earth metals;
basic compounds such as carbonates, bicarbonates, and organic acid salts of alkali metals and alkaline earth metals;
tertiary amines such as triethylamine, tributylamine, trihexylamine, and benzyldiethylamine;
nitrogen-containing heteroaromatic compounds such as N-alkylpyrrole, N-alkylindole, oxazole, N-alkylimidazole, N-alkylpyrazole, oxadiazole, pyridine, alkylpyridine, quinoline, alkylquinoline, isoquinoline, alkylisoquinoline, acridine, alkylacridine, phenanthroline, alkylphenanthroline, pyrimidine, alkylpyrimidine, pyrazine, alkylpyrazine, triazine, and alkyltriazine;
cyclic amidines such as diazabicycloundecene (DBU) and diazabicyclononene (DBN);
thallium compounds such as thallium oxide, thallium halide, thallium hydroxide, thallium carbonate, thallium nitrate,

thallium sulfate, and organic acid salts of thallium;
tin compounds such as tributylmethoxytin, tributylethoxytin, dibutyldimethoxytin, diethyldiethoxytin, dibutyldiethoxytin, dibutylphenoxytin, diphenylmethoxytin, dibutyltin acetate, tributyltin chloride, and tin 2-ethylhexanoate;
zinc compounds such as dimethoxyzinc, diethoxyzinc, ethylenedioxyzinc, and dibutoxyzinc;
aluminum compounds such as aluminum trimethoxide, aluminum triisopropoxide, and aluminum tributoxide;
titanium compounds such as tetramethoxytitanium, tetraethoxytitanium, tetrabutoxytitanium, dichlorodimethoxytitanium, tetraisopropoxytitanium, titanium acetate, and titanium acetylacetonate;
phosphorus compounds such as trimethylphosphine, triethylphosphine, tributylphosphine, triphenylphosphine, tributylmethyl phosphonium halide, trioctylbutyl phosphonium halide, and triphenylmethyl phosphonium halide;
zirconium compounds such as zirconium halide, zirconium acetylacetonate, zirconium alkoxide, and zirconium acetate;
lead and compounds containing lead, for example, lead oxides such as PbO, $PbO_2$, and $Pb_3O_4$;
lead sulfides such as PbS, $Pb_2S_3$, and $PbS_2$;
lead hydroxides such as $Pb(OH)_2$, $Pb_3O_2(OH)_2$, $Pb_2[PbO_2(OH)_2]$, and $Pb_2O(OH)_2$;
plumbites such as $Na_2PbO_2$, $K_2PbO_2$, $NaHPbO_2$, and $KHPbO_2$;
plumbates such as $Na_2PbO_3$, $Na_2H_2PbO_4$, $K_2PbO_3$, $K_2[Pb(OH)_6]$, $K_4PbO_4$, $Ca_2PbO_4$, and $CaPbO_3$;
carbonate of lead and basic salts thereof, such as $PbCO_3$ and $2PbCO_3 \cdot Pb(OH)_2$;
alkoxyleads and aryloxyleads such as $Pb(OCH_3)_2$, $(CH_3O)Pb(OPh)$, and $Pb(OPh)_2$;
lead salts of organic acids and carbonate and basic salts thereof, such as $Pb(OCOCH_3)_2$, $Pb(OCOCH_3)_4$, and $Pb(OCOCH_3)_2 \cdot PbO \cdot 3H_2O$;
organic lead compounds such as $Bu_4Pb$, $Ph_4Pb$, $Bu_3PbCl$, $Ph_3PbBr$, $Ph_3Pb$ (or $Ph_6Pb_2$), $Bu_3PbOH$, and $Ph_2PbO$ (Bu represents a butyl group, and Ph represents a phenyl group);
alloys of lead such as Pb-Na, Pb-Ca, Pb-Ba, Pb-Sn, and Pb-Sb; and
lead minerals such as galena and sphalerite, and hydrides of these lead compounds.

These compounds can be used as homogeneous catalysts when soluble in reaction raw materials, a reaction mixture, reaction by-products, or the like, and can be used as solid catalysts when insoluble therein. These compounds are dissolved beforehand in reaction raw materials, a reaction mixture, reaction by-products, or the like, or use of a mixture dissolved through a reaction as a homogeneous catalyst is also a preferred method.

Further examples of the catalyst used include: ion exchangers such as anion-exchange resins having a tertiary amino group, ion-exchange resins having an amide group, ion-exchange resins having at least one exchange group selected from a sulfonic acid group, a carboxylic acid group, and a phosphoric acid group, and solid strongly basic anion exchangers having a quaternary ammonium group as an exchange group; and solid inorganic compounds such as silica, silica-alumina, silica-magnesia, aluminosilicate, gallium silicate, various zeolites, various metal-exchanged zeolites, and ammonium-exchanged zeolites.

A solid strongly basic anion exchanger having a quaternary ammonium group as an exchange group is particularly preferably used as a solid catalyst. Examples of such a solid strongly basic anion exchanger include, but are not particularly limited to, strongly basic anion-exchange resins having a quaternary ammonium group as an exchange group, cellulose strongly basic anion exchangers having a quaternary ammonium group as an exchange group, and strongly basic anion exchangers of type supported on an inorganic support having a quaternary ammonium group as an exchange group. The strongly basic anion-exchange resin having a quaternary ammonium group as an exchange group is not particularly limited, and, for example, a styrenic strongly basic anion-exchange resin is preferably used. The styrenic strongly basic anion-exchange resin is a strongly basic anion-exchange resin having a copolymer of styrene and divinylbenzene as a matrix and having quaternary ammonium (type I or type II) as an exchange group, and is schematically represented by, for example, the following formula:

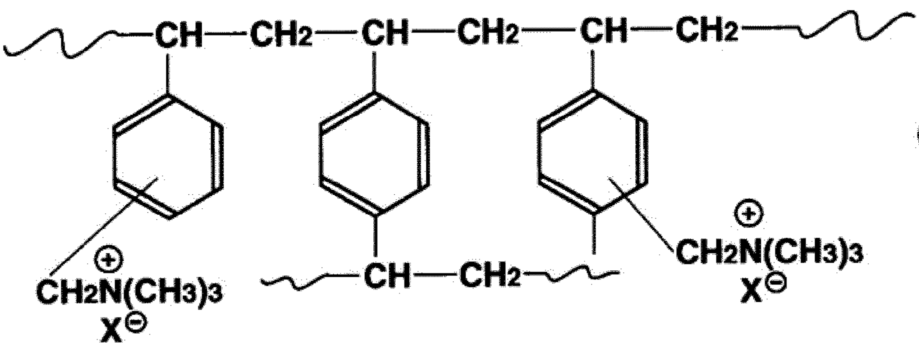

(type I)

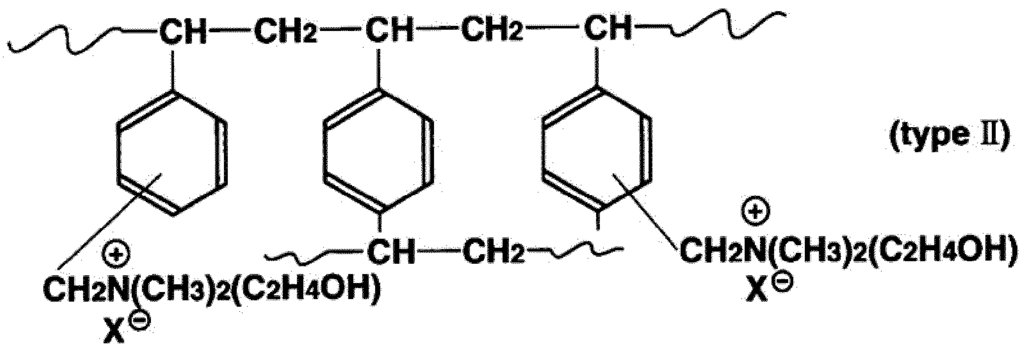

(type II)

In the formula, X represents an anion. At least one anion selected from among $F^-$, $Cl^-$, $Br^-$, $I^-$, $HCO_3^-$, $CO_3^{2-}$, $CH_3CO_2^-$, $HCO_2^-$, $IO_3^-$, $BrO_3^-$, and $ClO_3^-$ is usually used as X, and at least one anion selected from among Cl-, Br-, $HCO_3^-$, and $CO_3^{2-}$ is preferably used. Any of gel type and macroreticular type (MR type) can be used as the structure of the resin matrix, and MR type is particularly preferred from the viewpoint of high organic solvent resistance.

Examples of the cellulose strongly basic anion exchanger having a quaternary ammonium group as an exchange group include, but are not particularly limited to, cellulose having an exchange group $-OCH_2CH_2NR_3X$ obtained by trialkylaminoethylating some or all of -OH groups of cellulose. In the formula, R represents an alkyl group. Methyl, ethyl, propyl, butyl, or the like is usually used, and methyl or ethyl is preferably used. X represents an anion, as mentioned above.

The strongly basic anion exchanger of type supported on an inorganic support having a quaternary ammonium group as an exchange group means an anion exchanger in which a quaternary ammonium group - $O(CH_2)nNR_3X$ is introduced by modifying some or all of surface hydroxy groups -OH of an inorganic support. In the formula, R and X are as mentioned above. n is usually an integer of 1 to 6, preferably n = 2. The inorganic support is not particularly limited, and, for example, silica, alumina, silica-alumina, titania, or zeolite can be used. Silica, alumina, or silica-alumina is preferably used, and silica is particularly preferably used. An arbitrary method can be used as a method for modifying the surface hydroxy groups of an inorganic support.

A commercially available product can also be used as the solid strongly basic anion exchanger having a quaternary ammonium group as an exchange group. In this case, ion exchange may be performed with the desired anion species in pretreatment, and the resultant can then be used as a transesterification catalyst.

An organic polymer of macroreticular or gel type bonded to a heterocyclic group containing at least one nitrogen atom, or a solid catalyst formed from an inorganic support bonded to a heterocyclic group containing at least one nitrogen atom is also preferably used as a transesterification catalyst. Further, a solid catalyst in which some or all of such nitrogen-containing heterocyclic groups are converted into a quaternary salt is similarly used. A solid catalyst such as an ion exchanger can also function as a packing.

The amount of the catalyst used in the step (I) differs depending on the type of the catalyst used. In the case of continuously supplying a homogeneous catalyst soluble in a reaction liquid under reaction conditions, the catalyst is used at usually 0.0001 to 50% by mass, preferably 0.005 to 20% by mass, further preferably 0.01 to 10% by mass, in terms of a ratio to the total mass of the cyclic carbonate and the aliphatic monovalent alcohol serving as supplied raw materials. In the case of using a solid catalyst installed in the distillation column, the catalyst is preferably used in an amount of 0.01 to 75% by volume, preferably 0.05 to 60% by volume, further preferably 0.1 to 60% by volume, based on the vacant column volume of the distillation column.

In the step (I), the method for continuously supplying the raw materials cyclic carbonate and the aliphatic monovalent alcohol into a continuous multistage distillation column $T_0$ which is a reactive distillation column is not

particularly limited and can be any method as long as the supply method can bring the raw materials into contact with a catalyst, for example, in a region of at least five or more stages, preferably seven or more stages, more preferably ten or more stages, of the distillation column. Specifically, the cyclic carbonate and the aliphatic monovalent alcohol can be continuously supplied from a necessary number of introduction ports for stages that satisfy the conditions described above in the continuous multistage distillation column. The cyclic carbonate and the aliphatic monovalent alcohol may be introduced to the same stages of the distillation column or may be separately introduced to different stages.

The raw materials cyclic carbonate and aliphatic monovalent alcohol are continuously supplied in a liquid form, in a gas form, or as a mixture of a liquid and a gas into the continuous multistage distillation column $T_0$. In addition to such supply of the raw materials into the distillation column, the raw materials may be additionally supplied intermittently or continuously in a gas form from a lower portion of the distillation column, and this is also a preferred method. A method of continuously supplying the cyclic carbonate in a liquid form or in a gas-liquid mixed state to stages above stages containing a catalyst in the distillation column, and continuously supplying the aliphatic monovalent alcohol in a gas form and/or in a liquid form to a lower portion of the distillation column is also a preferred method. In this case, the cyclic carbonate may contain the aliphatic monovalent alcohol, as a matter of course.

In the step (I), the supplied raw materials may contain the products dialkyl carbonate and/or diols. The contents thereof are usually 0 to 40% by mass, preferably 0 to 30% by mass, further preferably 0 to 20% by mass, of the dialkyl carbonate in terms of % by mass in an aliphatic monovalent alcohol/dialkyl carbonate mixture, and usually 0 to 10% by mass, preferably 0 to 7% by mass, further preferably 0 to 5% by mass, of the diols in terms of % by mass in a cyclic carbonate/diol mixture.

In the case of industrially carrying out the reaction of the step (I), it is preferred that cyclic carbonate and/or aliphatic monovalent alcohols that are newly introduced to the reaction system as well as a substance composed mainly of cyclic carbonate and/or aliphatic monovalent alcohols recovered in this step and/or other steps should be able to be used as raw materials. Other steps include, for example, a step (II) of producing diaryl carbonate (e.g., diphenyl carbonate) from dialkyl carbonate and aryl alcohol (e.g., phenol), and in this step (II), aliphatic monovalent alcohols are recovered as by-products. The aliphatic monovalent alcohols recovered as by-products often contain dialkyl carbonate, aryl alcohol (e.g., phenol), alkyl aryl ether, and the like and may further contain a small amount of alkyl aryl carbonate, diaryl carbonate (e.g., diphenyl carbonate), and the like. The by-products aliphatic monovalent alcohols may be used directly as raw materials in the step (I) or may be used as raw materials in the step (I) after the content of substances having a higher boiling point than that of the aliphatic monovalent alcohols is decreased by distillation or the like.

The preferred cyclic carbonate for use in the step (I) is produced, for example, through the reaction of alkylene oxide such as ethylene oxide, propylene oxide, or styrene oxide with carbon dioxide. Therefore, cyclic carbonate containing a small amount of these compounds, etc. may be used as raw materials in the step (I).

In the step (I), the quantitative ratio between the cyclic carbonate and the aliphatic monovalent alcohols to be supplied to the reactive distillation column differs depending on the type and amount of a transesterification catalyst and reaction conditions. Usually, the aliphatic monovalent alcohols can be supplied in the range of 0.01 to 1000 times the mol of the cyclic carbonate to be supplied. For enhancing the reaction ratio of the cyclic carbonate, it is preferred to supply the aliphatic monovalent alcohols in an excessive amount of twice or more the mol of the cyclic carbonate, though use of the aliphatic monovalent alcohols in a large excess requires increasing the size of an apparatus. In this respect, the molar ratio of the aliphatic monovalent alcohols to the cyclic carbonate is preferably 2 to 20, more preferably 3 to 15, further preferably 5 to 12. If unreacted cyclic carbonate remains in a large amount, this cyclic carbonate reacts with the products diols to form multimers such as dimers or trimers as by-products. Therefore, in the case of carrying out an industrial process, it is preferred to minimize the amount of unreacted cyclic carbonate remaining.

In the step (I), for example, in the case of continuously producing approximately 0.4 tons or more of dialkyl carbonate per hour, the lowest amount of the cyclic carbonate continuously supplied is usually 0.44P ton/hr, preferably 0.42P ton/hr, more preferably 0.4P ton/hr, based on the amount (P ton/hr) of the dialkyl carbonate to be produced. Further preferably, the lowest amount can be smaller than 0.39P ton/hr.

The continuous multistage distillation column $T_0$ for use in the step (I) is not particularly limited and is, for example, a continuous multistage distillation column as shown in Figure 3 that has a cylindrical body with a length $L_0$ (cm) and an inside diameter $D_0$ (cm), has a structure having an internal having a number of stages $n_0$ in the inside, and has a gas discharge port with an inside diameter $d_{01}$ (cm) in a column top portion or an upper portion of the column close thereto, a liquid discharge port with an inside diameter $d_{02}$ (cm) in a column bottom portion or a lower portion of the column close thereto, one or more first introduction ports in an upper portion and/or an intermediate portion of the column below the gas discharge port, and one or more second introduction ports in an intermediate portion and/or a lower portion of the column above the liquid discharge port, wherein $L_0$, $D_0$, $L_0/D_0$, $n_0$, $D_0/d_{01}$, and $D_0/d_{02}$ respectively satisfy the following expressions (1) to (6) :

$$2100 \le L_0 \le 8000 \quad \text{... Expression (1)}$$

$$180 \leq D_0 \leq 2000 \ ... \ \text{Expression (2)}$$

$$4 \leq L_0/D_0 \leq 40 \ ... \ \text{Expression (3)}$$

$$10 \leq n_0 \leq 120 \ ... \ \text{Expression (4)}$$

$$3 \leq D_0/d_{01} \leq 20 \ ... \ \text{Expression (5)}$$

$$5 \leq D_0/d_{02} \leq 30 \ ... \ \text{Expression (6).}$$

The term "column top portion or upper portion of the column close thereto" used in the present embodiment means a portion from the column top portion to approximately $0.25L_0$ downward, and the term "column bottom portion or lower portion of the column close thereto" means a portion from the column bottom portion to approximately $0.25L_0$ upward (these correspond to $0.25L_1$ and $0.25L_2$, respectively, in the first and second continuous multistage distillation columns, and $0.25L_A$ and $0.25L_B$, respectively, in the purifying column A and the diphenyl carbonate purifying column B).

By using the continuous multistage distillation column $T_0$ that simultaneously satisfies the expressions (1), (2), (3), (4), (5), and (6), dialkyl carbonate and/or diols can be stably produced from cyclic carbonate and aliphatic monovalent alcohols with high reaction ratio, high selectivity, and high productivity at an industrial scale of preferably 0.4 tons or more of the dialkyl carbonate per hour and preferably 0.26 tons or more of the diols per hour for a long period of, for example, 1000 hours or longer, preferably 3000 hours or longer, further preferably 5000 hours or longer. The reason is not clear why the step (I) allows for the production of dialkyl carbonate and diols at an industrial scale having such excellent effects. The effects are presumably combined effects brought about by a combination of the conditions of the expressions (1) to (6). Preferred ranges of the respective factors will be given below.

When $L_0$ (cm) is 2100 or more, reaction ratio is favorable and the desired production amount can be achieved. For reducing the cost of equipment while securing the reaction ratio that can achieve the desired production amount, $L_0$ is preferably 8000 or less. $L_0$ (cm) is more preferably in the range of $2300 \leq L_0 \leq 6000$, further preferably $2500 \leq L_0 \leq 5000$.

When $D_0$ (cm) is 180 or more, the desired production amount can be achieved. For reducing the cost of equipment while achieving the desired production amount, $D_0$ is preferably 2000 or less. $D_0$ (cm) is more preferably in the range of $200 \leq D_0 \leq 1000$, further preferably $210 \leq D_0 \leq 800$.

When $L_0/D_0$ is 4 or more or is 40 or less, stable operation is easy to perform. Particularly, when $L_0/D_0$ is 40 or less, long-term stable operation is easy to perform because difference in pressure between the upper and lower portions of the column can be prevented from becoming too large. Besides, side reaction can be suppressed, and selectivity tends to be improved, because a temperature in the lower portion of the column can be decreased. $L_0/D_0$ is more preferably in the range of $5 \leq L_0/D_0 \leq 30$, further preferably $7 \leq L_0/D_0 \leq 20$.

When $n_0$ is 10 or more, the desired production amount can be achieved because reaction ratio is improved. For reducing the cost of equipment while securing the reaction ratio that can achieve the desired production amount, $n_0$ is preferably 120 or less. When $n_0$ is 120 or less, long-term stable operation is easy to perform because difference in pressure between the upper and lower portions of the column can be prevented from becoming too large. Besides, side reaction can be suppressed, and selectivity tends to be improved, because a temperature in the lower portion of the column can be decreased. $n_0$ is more preferably in the range of $30 \leq n_0 \leq 100$, further preferably $40 \leq n_0 \leq 90$.

When $D_0/d_{01}$ is 3 or more, the cost of equipment can be reduced. Besides, a gas component can be prevented from coming out of the system and stable operation is easy to perform. When the ratio is 20 or less, the amount of a gas component discharged is relatively large and stable operation is easy to perform. Besides reaction ratio tends to be improved. $D_0/d_{01}$ is more preferably in the range of $4 \leq D_0/d_{01} \leq 15$, further preferably $5 \leq D_0/d_{01} \leq 13$.

When $D_0/d_{02}$ is 5 or more, the cost of equipment can be reduced. Besides, the amount of a liquid discharged is relatively small and stable operation is easy to perform. When the ratio is 30 or less, a flow rate in a liquid discharge port or a piping can be prevented from being rapidly accelerated, erosion is unlikely to occur, and the corrosion of an apparatus can be suppressed. $D_0/d_{02}$ is more preferably in the range of $7 \leq D_0/d_{02} \leq 25$, further preferably $9 \leq D_0/d_{02} \leq 20$.

The factors $d_{01}$ and $d_{02}$ of the continuous multistage distillation column $T_0$ for use in the step (I) further preferably satisfy the expression (28):

$$1 \leq d_{01}/d_{02} \leq 5 \ ... \ \text{Expression (28).}$$

The long-term stable operation described in the step (I) means that operation can be continued for 1000 hours or longer, preferably 3000 hours or longer, further preferably 5000 hours or longer, in a steady state based on operation conditions without flooding or piping clogging or erosion, and predetermined amounts of dialkyl carbonate and diols are produced while high reaction ratio, high selectivity, and high productivity are maintained.

The selectivity of dialkyl carbonate and diols described in the step (I) is based on the reacted cyclic carbonate. In the present embodiment, high selectivity of usually 95% or more, preferably high selectivity of 97% or more, more preferably 99% or more, can be achieved. The reaction ratio described in the step (I) usually refers to the reaction ratio of the cyclic carbonate. In the present embodiment, the reaction ratio of the cyclic carbonate can be 95% or more, preferably 97% or more, more preferably 99% or more, further preferably 99.5 or more, still further preferably 99.9% or more. Such high reaction ratio that can be achieved with high selectivity maintained is also one of the excellent features of the step (I).

The continuous multistage distillation column $T_0$ for use in the step (I) is preferably a distillation column having a tray and/or a packing as the internal. The internal described in the present embodiment means a portion where the contact between a gas and a liquid is actually performed in the distillation column. Such a tray is not particularly limited and is preferably, for example, a bubble-cap tray, a sieve tray, a valve tray, a countercurrent tray, a Superfrac tray, or a Max-Frac tray, and the packing is preferably a random packing such as a Raschig ring, a Lessing ring, a pall ring, a Berl saddle, an Intalox saddle, Dixon Packing, Mc MAHON Packing, or Heli Pack, or a structured packing such as Mellapak, Gempak, Techno Pack, Flexipac, Sulzer Packings, Goodloe Packing, or Glitsch Grid. A multistage distillation column having both of a tray portion and a portion filled with a packing can also be used. The term "number of stages of the internal" described in the present embodiment means the number of trays in the case of a tray and means the theoretical number of stages in the case of a packing. Thus, in the case of a multistage distillation column having both of a tray portion and a portion filled with a packing, the number of stages is the total sum of the number of trays and the theoretical number of stages.

In the step (I) of reacting cyclic carbonate with aliphatic monovalent alcohols, use of any of a continuous multistage distillation column of plate column type and a continuous multistage distillation column of packed column type having a tray and/or a packing having a predetermined number of stages in the internal tends to be able to achieve high reaction ratio, high selectivity, and high productivity. The distillation column of plate column type having a tray in the internal is more preferred. The tray is a sieve tray having a sieve portion and a downcomer portion because of an excellent relationship between functions and the cost of equipment. The sieve tray preferably has 100 to 1000 holes per $m^2$ of the area of the sieve portion. The number of holes is more preferably 120 to 900, further preferably 150 to 800, per $m^2$ of the area. The cross section per hole of the sieve tray is preferably 0.5 to 5 $cm^2$. The cross section per hole is more preferably 0.7 to 4 $cm^2$, further preferably 0.9 to 3 $cm^2$. The case is particularly preferred where the sieve tray has 100 to 1000 holes per $m^2$ of the area of the sieve portion, and the cross section per hole is 0.5 to 5 $cm^2$.

The aperture ratio of the sieve tray is preferably 1.5 to 15%. The aperture ratio is more preferably 1.7 to 13%, further preferably 1.9 to 11%. In this context, the aperture ratio of the sieve tray refers to the ratio of the cross section of all holes (total hole cross section) present in a sieve to the area (including the total hole cross section) of the sieve portion. The area and/or total hole cross section of the sieve portion may differ among sieve trays. In this case as well, the aperture ratio of each sieve tray preferably falls within the range described above. The number of holes in the sieve portion may be the same among all sieves or may be different.

The step (I), when carried out, involves, for example, continuously supplying the raw materials cyclic carbonate and aliphatic monovalent alcohols into a continuous multistage distillation column containing a catalyst, simultaneously performing a reaction and distillation in the column, continuously discharging a low-boiling reaction mixture containing dialkyl carbonate thus formed in a gas form from an upper portion of the column, and continuously discharging a high-boiling reaction mixture containing diols in a liquid form from a lower portion of the column to continuously produce dialkyl carbonate and diols.

In the step (I), the raw materials cyclic carbonate and aliphatic monovalent alcohols, when continuously supplied into the continuous multistage distillation column $T_0$, may be supplied in a liquid form and/or in a gas form either as a raw material mixture or separately from an introduction port installed at one or several locations below an upper gas discharge port of the distillation column and in an upper portion or an intermediate portion of the column, or cyclic carbonate or a raw material rich therein may be supplied in a liquid form from an introduction port in an upper portion or an intermediate portion of the distillation column, while aliphatic monovalent alcohols or a raw material rich therein may be supplied in a gas form from an introduction port installed above a lower liquid discharge port of the distillation column and in an intermediate portion or a lower portion of the column, and this is also a preferred method.

The reaction time of the transesterification reaction that is performed in the step (I) presumably corresponds to an average residence time of a reaction liquid in the continuous multistage distillation column $T_0$. This differs depending on the shape and number of stages of the internal of the distillation column, the amount of raw materials supplied, the type and amount of a catalyst, reaction conditions, etc. and is usually 0.1 to 20 hours, preferably 0.5 to 15 hours, more preferably 1 to 10 hours.

The reaction temperature of the step (I) differs depending on the type of the raw material compound used and the type and amount of a catalyst, and is usually 30 to 300°C. Although a higher reaction temperature is preferred for enhancing a reaction rate, such a high reaction temperature easily causes side reaction. The reaction temperature is in the range of preferably 40 to 250°C, more preferably 50 to 200°C, further preferably 60 to 150°C. In the present embodiment, reactive distillation can be carried out at a column bottom temperature of 150°C or lower, preferably 130°C or lower, more preferably 110°C or lower, further preferably 100°C or lower. High reaction ratio, high selectivity, and high productivity that can be achieved even at such a low column bottom temperature is also one of the excellent features of the step (I). The reaction pressure differs depending on the type and composition of the raw material compound used, the reaction temperature, etc., and can be any of reduced pressure, normal pressure, and increased pressure. Usually, the reaction is performed in the range of 1 Pa to $2 \times 10^7$ Pa, preferably $10^3$ Pa to $10^7$ Pa, more preferably $10^4$ Pa to $5 \times 10^6$ Pa.

The reflux ratio for use in the continuous multistage distillation column $T_0$ in the step (I) is usually 0 to 10, preferably 0.01 to 5, more preferably 0.05 to 3.

The material constituting the continuous multistage distillation column $T_0$ for use in the step (I) is mainly a metal material such as carbon steel or stainless steel, and stainless steel is preferred from the viewpoint of the quality of the produced dialkyl carbonate and diols.

The method for industrially producing high-purity diaryl carbonate according to the present embodiment preferably further comprises the steps of: continuously supplying dialkyl carbonate and aryl alcohol into a first continuous multistage distillation column containing a catalyst, simultaneously performing a reaction and distillation in the first continuous multistage distillation column, continuously discharging a first column low-boiling reaction mixture containing alcohols thus formed in a gas form from an upper portion of the first continuous multistage distillation column, and continuously discharging a first column high-boiling reaction mixture containing alkyl aryl carbonate thus formed in a liquid form from a lower portion of the first continuous multistage distillation column to continuously produce alkyl aryl carbonate; and continuously supplying the first column high-boiling reaction mixture into a second continuous multistage distillation column containing a catalyst, simultaneously performing a reaction and distillation in the second continuous multistage distillation column, continuously discharging a second column low-boiling reaction mixture containing dialkyl carbonates thus formed in a gas form from an upper portion of the second continuous multistage distillation column, and continuously discharging a second column high-boiling reaction mixture containing diaryl carbonate thus formed in a liquid form from a lower portion of the second continuous multistage distillation column to continuously produce diaryl carbonate (hereinafter, these two steps are also collectively referred to as a "step (II)").

Examples of the dialkyl carbonate for use in the step (II) include, but are not particularly limited to, compounds represented by the following formula described in Formula 1 described above:

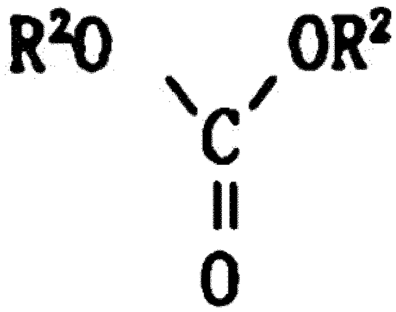

In the formula, $R^2$ is as described above.

Examples of the dialkyl carbonate having such $R^2$ include, but are not particularly limited to, dimethyl carbonate, diethyl carbonate, dipropyl carbonate (each isomer), diallyl carbonate, dibutenyl carbonate (each isomer), dibutyl carbonate (each isomer), dipentyl carbonate (each isomer), dihexyl carbonate (each isomer), diheptyl carbonate (each isomer), dioctyl carbonate (each isomer), dinonyl carbonate (each isomer), didecyl carbonate (each isomer), dicyclopentyl carbonate, dicyclohexyl carbonate, dicycloheptyl carbonate, dibenzyl carbonate, diphenethyl carbonate (each isomer), di(phenylpropyl) carbonate (each isomer), di(phenylbutyl) carbonate (each isomer), di(chlorobenzyl) carbonate (each isomer), di(methoxybenzyl) carbonate (each isomer), di(methoxymethyl) carbonate, di(methoxyethyl) carbonate (each isomer), di(chloroethyl) carbonate (each isomer), and di(cyanoethyl) carbonate (each isomer).

Among them, dialkyl carbonate wherein $R^2$ is an alkyl group having 4 or less carbon atoms and containing no halogen is preferably used in the present embodiment, and dimethyl carbonate is particularly preferred. The preferred dialkyl carbonate is further preferably dialkyl carbonate produced in a state substantially free of halogen, and is produced from, for example, alkylene carbonate substantially free of halogen and an alcohol substantially free of halogen.

The phenol for use in the step (II) is represented by the following general formula and is a compound in which

a hydroxyl group is bonded directly to a phenyl group (Ph), though substituted phenol in which a phenyl group is substituted by a lower alkyl group or a lower alkoxy group may be used:

PhOH

In the present embodiment, phenol substantially free of halogen is preferably used. Thus, the diphenyl carbonate described in the present embodiment is represented by the following general formula:

PhOCOOPh

The molar ratio of the dialkyl carbonate to the aryl alcohol (e.g., phenol) for use as raw materials in the step (II) is preferably 0.1 to 10. When the molar ratio falls within this range, unreacted raw materials remain in a small amount with respect to a predetermined production amount of the desired diphenyl carbonate, which are efficient. Also, energy for recovering these unreacted materials can be suppressed. In this respect, the molar ratio is more preferably 0.5 to 5, further preferably 0.8 to 3, still further preferably 1 to 2.

In the present embodiment, it is preferred to continuously produce 1 ton or more of high-purity diaryl carbonate (e.g., diphenyl carbonate) per hour.

Thus, in the step (II), the lowest amount of the aryl alcohol (e.g., phenol) continuously supplied is usually 15Q ton/hr, preferably 13Q ton/hr, more preferably 10Q ton/hr, based on the amount (Q ton/hr) of the high-purity diaryl carbonate (e.g., diphenyl carbonate) to be produced. Further preferably, the lowest amount can be smaller than 8Q ton/hr.

Each of the dialkyl carbonate and the aryl alcohol (e.g., phenol) for use as raw materials in the step (II) may have a high purity or may contain other compounds. The raw materials may contain, for example, compounds and reaction by-products formed in the first continuous multistage distillation column and/or the second continuous multistage distillation column. In the case of carrying out an industrial process, dialkyl carbonate and aryl alcohol (e.g., phenol) that are newly introduced into the reaction system as well as those recovered from the first continuous multistage distillation column and/or the second continuous multistage distillation column are preferably used as these raw materials. In the method of the present embodiment, a first circulation step of continuously supplying a column top component which is a second column low-boiling reaction mixture in the second continuous multistage distillation column into the first continuous multistage distillation column is performed. In this case, the second column low-boiling reaction mixture may be supplied directly into the first continuous multistage distillation column or may be supplied after a portion of the component is separated.

In the present embodiment to be industrially carried out, the raw materials to be supplied into the first continuous multistage distillation column preferably contain alcohols, alkyl aryl carbonate (e.g., alkyl phenyl carbonate), diaryl carbonate (e.g., diphenyl carbonate), alkyl aryl ether (e.g., alkyl phenyl ether), and the like, and even raw materials further containing a small amount of high-boiling by-products such as Fries rearrangement products of the product aryl carbonate (e.g., phenyl carbonate) or diaryl carbonate (e.g., diphenyl carbonate) or their derivatives are preferably used. In the present embodiment, for example, in the case of producing methyl phenyl carbonate and diphenyl carbonate by using dimethyl carbonate as the dialkyl carbonate and unsubstituted phenol as the phenol as raw materials, the raw materials preferably contain the reaction products methyl alcohol, methyl phenyl carbonate, and diphenyl carbonate and may further contain a small amount of reaction by-products anisole, phenyl salicylate, and methyl salicylate or high-boiling by-products derived therefrom.

The diaryl carbonate (e.g., diphenyl carbonate) obtained by the production method of the present embodiment is preferably used for producing aromatic polycarbonate through polymerization reaction with an aromatic dihydroxy compound. In this polymerization step, a large amount of aryl alcohol (e.g., phenol) is formed as by-products and recovered. Use of this aryl alcohol (e.g., phenol) as by-products as a raw material of the step (II) is also a preferred method.

The diaryl carbonate (e.g., diphenyl carbonate) to be produced in the step (II) is obtained through transesterification reaction of dialkyl carbonate and aryl alcohol (e.g., phenol). This transesterification reaction includes a reaction of exchanging one or two alkoxy groups of the dialkyl carbonate with an alkoxy group (e.g., a phenoxy group) of the aryl alcohol (e.g., phenol) to eliminate alcohols, and a reaction of converting the formed alkyl aryl carbonate (e.g., alkyl phenyl carbonate) into diaryl carbonate (e.g., diphenyl carbonate) and dialkyl carbonate through disproportionation reaction which is transesterification reaction between two molecules of the alkyl aryl carbonate. In the first continuous multistage distillation column of the step (II), alkyl aryl carbonate (e.g., alkyl phenyl carbonate) is mainly obtained, and in the second continuous multistage distillation column, diaryl carbonate (e.g., diphenyl carbonate) and dialkyl carbonate are mainly obtained through the disproportionation reaction of this alkyl aryl carbonate (e.g., alkyl phenyl carbonate). The diaryl carbonate (e.g., diphenyl carbonate) obtained in the step (II) is preferably totally free of halogen. Such diaryl carbonate (e.g., diphenyl carbonate) is useful, for example, as a raw material in industrially producing aromatic polycarbonate. This is because the presence of halogen even in an amount smaller than 1 ppm, for example, in polymerization raw materials inhibits polymerization reaction, inhibits stable production of aromatic polycarbonate, and causes reduction in the physical

properties of the formed aromatic polycarbonate and stain.

The catalyst for use in the first continuous multistage distillation column and/or the second continuous multistage distillation column in the step (II) is not particularly limited and is selected from, for example, the following compounds:

A metal-containing compound is used as the catalyst, for example:

<lead compounds>

lead oxides such as PbO, $PbO_2$, and $Pb_3O_4$;
lead sulfides such as PbS and $Pb_2S$;
lead hydroxides such as $Pb(OH)_2$ and $Pb_2O_2(OH)_2$;
plumbites such as $Na_2PbO_2$, $K_2PbO_2$, $NaHPbO_2$, and $KHPbO_2$;
plumbates such as $Na_2PbO_3$, $Na_2H_2PbO_4$, $K_2PbO_3$, $K_2[Pb(OH)_6]$, $K_4PbO_4$, $Ca_2PbO_4$, and $CaPbO_3$;
carbonate of lead and basic salts thereof, such as $PbCO_3$ and $2PbCO_3{\cdot}Pb(OH)_2$;
lead salts of organic acids and carbonate and basic salts thereof, such as $Pb(OCOCH_3)_2$, $Pb(OCOCH_3)_4$, and $Pb(OCOCH_3)_2{\cdot}PbO{\cdot}3H_2O$;
organic lead compounds such as $Bu_4Pb$, $Ph_4Pb$, $Bu_3PbCl$, $Ph_3PbBr$, $Ph_3Pb$ (or $Ph_6Pb_2$), $Bu_3PbOH$, and $Ph_3PbO$ (Bu represents a butyl group, and Ph represents a phenyl group);
alkoxyleads and aryloxyleads such as $Pb(OCH_3)_2$, $(CH_3O)Pb(OPh)$, and $Pb(OPh)_2$;
alloys of lead such as Pb-Na, Pb-Ca, Pb-Ba, Pb-Sn, and Pb-Sb; and
lead minerals such as galena and sphalerite, and hydrides of these lead compounds;

<copper family metal compounds>
salts and complexes of copper family metals such as CuCl, $CuCl_2$, CuBr, $CuBr_2$, CuI, $CuI_2$, $Cu(OAc)_2$, $Cu(acac)_2$, copper oleate, $Bu_2Cu$, $(CH_3O)_2Cu$, $AgNO_3$, AgBr, silver picrate, $AgC_6H_6ClO_4$, $[AuC{\equiv}C\text{-}C(CH_3)_3]n$, and $[Cu(C_7H_8)Cl]_4$ (acac represents an acetylacetone chelating ligand);
<alkali metal complexes>
alkali metal complexes such as Li(acac) and $LiN(C_4H_9)_2$;
<zinc complexes>
zinc complexes such as $Zn(acac)_2$;
<cadmium complexes>
cadmium complexes such as $Cd(acac)_2$;
<iron family metal compounds>
iron family metal complexes such as $Fe(C_{10}H_8)(CO)_5$, $Fe(CO)_5$, $Fe(C_4H_6)(CO)_3$, $Co(mesitylene)_2(PEt_2Ph)_2$, $CoC_5F_5(CO)_7$, Ni-$\pi$-$C_5H_5NO$, and ferrocene;
<zirconium complexes>
zirconium complexes such as $Zr(acac)_4$ and zirconocene;
<Lewis acid compounds>
Lewis acids and transition metal compounds that generate Lewis acids, such as $AlX_3$, $TiX_3$, $TiX_4$, $VOX_3$, $VX_5$, $ZnX_2$, $FeX_3$, and $SnX_4$ (wherein X is halogen, an acetoxy group, an alkoxy group, or an aryloxy group); and
<organic tin compounds>
organic tin compounds such as $(CH_3)_3SnOCOCH_3$, $(C_2H_5)_3SnOCOC_6H_5$, $Bu_3SnOCOCH_3$, $Ph_3SnOCOCH_3$, $Bu_2Sn(OCOCH_3)_2$, $Bu_2Sn(OCOC_{11}H_{23})_2$, $Ph_3SnOCH_3$, $(C_2H_5)_3SnOPh$, $Bu_2Sn(OCH_3)_2$, $Bu_2Sn(OC_2H_5)_2$, $Bu_2Sn(OPh)_2$, $Ph_2Sn(OCH_3)_2$, $(C_2H_5)_3SnOH$, $Ph_3SnOH$, $Bu_2SnO$, $(C_8H_{17})_2SnO$, $Bu_2SnCl_2$, and BuSnO(OH).

The catalyst used in the present embodiment preferably contains a metal such as Pb, Cu, Zn, Fe, Co, Ni, Al, Ti, V, or Sn and is a homogeneous catalyst soluble in in the reaction system. Thus, a catalyst species in which such a metal component is bonded to an organic group is preferably used. As a matter of course, these catalytic components may be reacted with organic compounds present in the reaction system, for example, aliphatic alcohols, phenols, alkyl phenyl carbonates, diphenyl carbonates, or dialkyl carbonates, or may be heat-treated with raw materials or products prior to the reaction. The catalyst used in the present embodiment preferably has a high solubility in a reaction liquid under reaction conditions. In this respect, preferred examples of the catalyst include: PbO, $Pb(OH)_2$ and $Pb(OPh)_2$; $TiCl_4$, $Ti(OMe)_4$, $(MeO)Ti(OPh)_3$, $(MeO)_2Ti(OPh)_2$, $(MeO)_3Ti(OPh)$, and $Ti(OPh)_4$; $SnCl_4$, $Sn(OPh)_4$, $Bu_2SnO$, and $Bu_2Sn(OPh)_2$; $FeCl_3$, $Fe(OH)_3$, and $Fe(OPh)_3$; and these catalysts treated with phenol or a reaction liquid. The catalyst for use in the first continuous multistage distillation column and the catalyst for use in the second continuous multistage distillation column may be of the same type or may be of different types.

In the present embodiment, it is particularly preferred to use halogen-free raw materials and a catalyst. In this case, the produced diaryl carbonate (e.g., diphenyl carbonate) is totally free of halogen and is therefore useful as a raw

material in industrially producing polycarbonate by a transesterification method. This is because the presence of halogen even in an amount smaller than 1 ppm, for example, in polymerization raw materials inhibits polymerization reaction, reduces the physical properties of the formed polycarbonate, and causes stain.

The first continuous multistage distillation column for use in the step (II) is preferably, for example, a continuous multistage distillation column as shown in Figure 4 that has a cylindrical body with a length $L_1$ (cm) and an inside diameter $D_1$ (cm), has a structure having an internal having a number of stages $n_1$ in the inside, and has a gas discharge port with an inside diameter $d_{11}$ (cm) in a column top portion or an upper portion of the column close thereto, a liquid discharge port with an inside diameter $d_{12}$ (cm) in a column bottom portion or a lower portion of the column close thereto, one or more third introduction ports in an upper portion and/or an intermediate portion of the column below the gas discharge port, and one or more fourth introduction ports in an intermediate portion and/or a lower portion of the column above the liquid discharge port, wherein $L_1$, $D_1$, $L_1/D_1$, $n_1$, $D_1/d_{11}$, and $D_1/d_{12}$ respectively satisfy the following expressions (7) to (12):

$$1500 \le L_1 \le 8000 \quad \text{... Expression (7)}$$

$$100 \le D_1 \le 2000 \quad \text{... Expression (8)}$$

$$2 \le L_1/D_1 \le 40 \quad \text{... Expression (9)}$$

$$20 \le n_1 \le 120 \quad \text{... Expression (10)}$$

$$5 \le D_1/d_{11} \le 30 \quad \text{... Expression (11)}$$

$$3 \le D_1/d_{12} \le 20 \quad \text{... Expression (12)}$$

The second continuous multistage distillation column for use in the step (II) is preferably, for example, a continuous multistage distillation column as shown in Figure 5 that has a cylindrical body with a length $L_2$ (cm) and an inside diameter $D_2$ (cm), has a structure having an internal having a number of stages $n_2$ in the inside, and has a gas discharge port with an inside diameter $d_{21}$ (cm) in a column top portion or an upper portion of the column close thereto, a liquid discharge port with an inside diameter $d_{22}$ (cm) in a column bottom portion or a lower portion of the column close thereto, one or more fifth introduction ports in an upper portion and/or an intermediate portion of the column below the gas discharge port, and one or more sixth introduction ports in an intermediate portion and/or a lower portion of the column above the liquid discharge port, wherein $L_2$, $D_2$, $L_2/D_2$, $n_2$, $D_2/d_{21}$, and $D_2/d_{22}$ respectively satisfy the following expressions (13) to (18):

$$1500 \le L_2 \le 8000 \quad \text{... Expression (13)}$$

$$100 \le D_2 \le 2000 \quad \text{... Expression (14)}$$

$$2 \le L_2/D_2 \le 40 \quad \text{... Expression (15)}$$

$$10 \le n_2 \le 80 \quad \text{... Expression (16)}$$

$$2 \le D_2/d_{21} \le 15 \quad \text{... Expression (17)}$$

$$5 \le D_2/d_{22} \le 30 \quad \text{... Expression (18)}$$

By using the first continuous multistage distillation column and the second continuous multistage distillation column that simultaneously satisfy the expressions (7) to (18), diaryl carbonate (e.g., diphenyl carbonate) tends to be able to be stably produced from dialkyl carbonate and aryl alcohol (e.g., phenol) with high selectivity and high productivity at an industrial scale of 0.5 tons or more, preferably 1 ton or more, per hour for a long period of, for example, 2000 hours or longer, preferably 3000 hours or longer, more preferably 5000 hours or longer. The reason is not clear why these distillation columns allow for the production of aromatic carbonate having such excellent effects at an industrial scale. The effects are presumably combined effects brought about by a combination of the conditions of the expressions (7) to (18). Preferred ranges of the respective factors constituting the continuous multistage distillation columns for use in the step (II) will be given below.

When each of $L_1$ (cm) and $L_2$ (cm) is 1500 or more, the desired production amount can be achieved because reaction ratio is improved. For reducing the cost of equipment while securing the reaction ratio that can achieve the desired production amount, each of $L_1$ and $L_2$ is preferably 8000 or less. $L_1$ (cm) and $L_2$ (cm) are more preferably in the ranges of $2000 \leq L_1 \leq 6000$ and $2000 \leq L_2 \leq 6000$, respectively, further preferably $2500 \leq L_1 \leq 5000$ and $2500 \leq L_2 \leq 5000$, respectively.

When each of $D_1$ (cm) and $D_2$ (cm) is 100 or more, the desired production amount can be achieved. For reducing the cost of equipment while achieving the desired production amount, each of $D_1$ and $D_2$ is preferably 2000 or less. $D_1$ (cm) and $D_2$ (cm) are more preferably in the ranges of $150 \leq D_1 \leq 1000$ and $150 \leq D_2 \leq 1000$, respectively, further preferably $200 \leq D_1 \leq 800$ and $200 \leq D_2 \leq 800$, respectively.

The first continuous multistage distillation column and the second continuous multistage distillation column may have the same inside diameter from the respective upper to lower portions of the columns or may have partially different inside diameters as long as $D_1$ and $D_2$ fall within the ranges described above. In these continuous multistage distillation columns, for example, the upper inside diameters of the columns may be smaller or larger than the lower inside diameters of the columns.

When each of $L_1/D_1$ and $L_2/D_2$ is 2 or more or is 40 or less, stable operation is easy to perform. Particularly, when each of the ratios is 40 or less, long-term stable operation is easy to perform because difference in pressure between the upper and lower portions of the column can be prevented from becoming too large. Besides, side reaction can be suppressed, and selectivity tends to be improved, because a temperature in the lower portion of the column can be decreased. $L_1/D_1$ and $L_2/D_2$ are more preferably in the ranges of $3 \leq L_1/D_1 \leq 30$ and $3 \leq L_2/D_2 \leq 30$, respectively, further preferably $5 \leq L_1/D_1 \leq 15$ and $5 \leq L_2/D_2 \leq 15$, respectively.

When $n_1$ is 20 or more, the desired production amount in the first continuous multistage distillation column can be achieved because reaction ratio is improved. For reducing the cost of equipment while securing the reaction ratio that can achieve the desired production amount, $n_1$ is preferably 120 or less. When $n_1$ is 120 or less, long-term stable operation of the first continuous multistage distillation column is easy to perform because difference in pressure between the upper and lower portions of the column can be prevented from becoming too large. Besides, side reaction can be suppressed, and selectivity tends to be improved, because a temperature in the lower portion of the column can be decreased. $n_1$ is more preferably in the range of $30 \leq n_1 \leq 100$, further preferably $40 \leq n_1 \leq 90$.

When $n_2$ is 10 or more, the desired production amount in the second continuous multistage distillation column can be achieved because reaction ratio is improved. For reducing the cost of equipment while securing the reaction ratio that can achieve the desired production amount, $n_2$ is preferably 80 or less. When $n_2$ is 80 or less, long-term stable operation of the second continuous multistage distillation column is easy to perform because difference in pressure between the upper and lower portions of the column can be prevented from becoming too large. Besides, side reaction can be suppressed, and selectivity tends to be improved, because a temperature in the lower portion of the column can be decreased. $n_2$ is more preferably in the range of $15 \leq n_2 \leq 60$, further preferably $20 \leq n_2 \leq 50$.

When $D_1/d_{11}$ is 5 or more, the cost of equipment of the first continuous multistage distillation column can be reduced. Stable operation of the first continuous multistage distillation column is easy to perform because a gas component can be prevented from coming out of the system. When the ratio is 30 or less, the amount of a gas component discharged is relatively large and stable operation is easy to perform. Besides, reaction ratio tends to be improved. $D_1/d_{11}$ is more preferably in the range of $8 \leq D_1/d_{11} \leq 25$, further preferably $10 \leq D_1/d_{11} \leq 20$. When $D_2/d_{21}$ is 2 or more, the cost of equipment of the second continuous multistage distillation column can be reduced. Stable operation of the second continuous multistage distillation column is easy to perform because a gas component can be prevented from coming out of the system. When the ratio is 15 or less, the amount of a gas component discharged is relatively large and stable operation is easy to perform. Besides, reaction ratio tends to be improved. $D_2/d_{21}$ is more preferably in the range of $5 \leq D_2/d_{21} \leq 12$, further preferably $3 \leq D_2/d_{21} \leq 10$.

When $D_1/d_{12}$ is 3 or more, the cost of equipment of the first continuous multistage distillation column can be reduced. The amount of a liquid discharged is relatively small, and stable operation of the first continuous multistage distillation column is easy to perform. When the ratio is 20 or less, a flow rate in a liquid discharge port or a piping can be prevented from being rapidly accelerated, erosion is unlikely to occur, and the corrosion of an apparatus can be suppressed. $D_1/d_{12}$ is more preferably in the range of $5 \leq D_1/d_{12} \leq 18$, further preferably $7 \leq D_1/d_{12} \leq 15$. When $D_2/d_{22}$

is 5 or more, the cost of equipment of the second continuous multistage distillation column can be reduced. The amount of a liquid discharged is relatively small, and stable operation of the second continuous multistage distillation column is easy to perform. When the ratio is 30 or less, a flow rate in a liquid discharge port or a piping can be prevented from being rapidly accelerated, erosion is unlikely to occur, and the corrosion of an apparatus can be suppressed. $D_2/d_{22}$ is more preferably in the range of $7 \leq D_2/d_{22} \leq 25$, further preferably $9 \leq D_2/d_{22} < 20$.

In the step (II), the factors $d_{11}$ and $d_{12}$ further preferably satisfy the expression (29), and the factors $d_{21}$ and $d_{22}$ further preferably satisfy the expression (30) :

$$1 \leq d_{12}/d_{11} \leq 5 \text{ ... Expression (29)}$$

$$1 \leq d_{21}/d_{22} \leq 6 \text{ ... Expression (30)}$$

The long-term stable operation described in the step (II) means that operation can be continued for 1000 hours or longer, preferably 3000 hours or longer, further preferably 5000 hours or longer, in a steady state based on operation conditions without flooding, weeping, piping clogging or erosion, or the like, and a predetermined amount of diaryl carbonate (e.g., diphenyl carbonate) is produced while high selectivity is maintained.

In the step (II), diaryl carbonate (e.g., diphenyl carbonate) is stably produced with high selectivity for a long period at high productivity of preferably 1 ton or more per hour, and more preferably 2 tons or more, further preferably 3 tons or more, of diphenyl carbonate per hour are produced. In the step (II), when $L_1$, $D_1$, $L_1/D_1$, $n_1$, $D_1/d_{11}$, and $D_1/d_{12}$ of the first continuous multistage distillation column satisfy $2000 \leq L_1 \leq 6000$, $150 \leq D_1 \leq 1000$, $3 \leq L_1/D_1 \leq 30$, $30 \leq n_1 \leq 100$, $8 \leq D_1/d_{11} \leq 25$, and $5 \leq D_1/d_{12} \leq 18$, respectively, and $L_2$, $D_2$, $L_2/D_2$, $n_2$, $D_2/d_{21}$, and $D_2/d_{22}$ of the second continuous multistage distillation column satisfy $2000 \leq L_2 \leq 6000$, $150 \leq D_2 \leq 1000$, $3 \leq L_2/D_2 \leq 30$, $15 \leq n_2 \leq 60$, $2.5 \leq D_2/d_{21} \leq 12$, and $7 \leq D_2/d_{22} \leq 25$, respectively, 2 tons or more, preferably 2.5 tons or more, further preferably 3 tons or more, of diaryl carbonate (e.g., diphenyl carbonate) per hour are produced.

In the step (II), when $L_1$, $D_1$, $L_1/D_1$, $n_1$, $D_1/d_{11}$, and $D_1/d_{12}$ of the first continuous multistage distillation column satisfy $2500 \leq L_1 \leq 5000$, $200 \leq D_1 \leq 800$, $5 \leq L_1/D_1 \leq 15$, $40 \leq n_1 \leq 90$, $10 \leq D_1/d_{11} \leq 25$, and $7 \leq D_1/d_{12} \leq 15$, respectively, and $L_2$, $D_2$, $L_2/D_2$, $n_2$, $D_2/d_{21}$, and $D_2/d_{22}$ of the second continuous multistage distillation column satisfy $2500 \leq L_2 \leq 5000$, $200 \leq D_2 \leq 800$, $5 \leq L_2/D_2 \leq 10$, $20 \leq n_2 \leq 50$, $3 \leq D_2/d_{21} \leq 10$, and $9 \leq D_2/d_{22} \leq 20$, respectively, 3 tons or more, preferably 3.5 tons or more, further preferably 4 tons or more, of diaryl carbonate (e.g., diphenyl carbonate) per hour are produced.

The selectivity of diaryl carbonate (e.g., diphenyl carbonate) described in the step (II) is based on the reacted aryl alcohol (e.g., phenol). In the step (II), high selectivity of usually 95% or more, preferably high selectivity of 97% or more, more preferably 99% or more, can be achieved.

Each of the first continuous multistage distillation column and the second continuous multistage distillation column for use in the step (II) is preferably a distillation column having a tray and/or a packing as the internal. The internal described in the present embodiment means a portion where the contact between a gas and a liquid is actually performed in each distillation column. Such a tray is preferably the tray described in the section of the step (I). The "number of stages of the internal" is as described above.

In the first continuous multistage distillation column of the step (II), a reaction of forming alkyl aryl carbonate (e.g., alkyl phenyl carbonate) from dialkyl carbonate and phenol is mainly performed. This reaction has a very small equilibrium constant and furthermore, has a slow reaction rate. Therefore, the first continuous multistage distillation column for use in reactive distillation is more preferably a distillation column of plate column type having a tray in the internal. In the second continuous multistage distillation column, a reaction of disproportioning the alkyl aryl carbonate (e.g., alkyl phenyl carbonate) is mainly performed. This reaction also has a very small equilibrium constant and furthermore, has a slow reaction rate. However, the second continuous multistage distillation column for use in reactive distillation is more preferably a distillation column having both a packing and a tray in the internal. In the second continuous multistage distillation column, the packing is preferably installed in an upper portion, and the tray is preferably installed in a lower portion. The packing of the second continuous multistage distillation column is preferably a structured packing, and the structured packing is particularly preferably Mellapak.

The tray to be installed in each of the first continuous multistage distillation column and the second continuous multistage distillation column is a sieve tray having a sieve portion and a downcomer portion because of an excellent relationship between functions and the cost of equipment in particular. The sieve tray preferably has 100 to 1000 holes per $m^2$ of the area of the sieve portion. The number of holes is more preferably 120 to 900, further preferably 150 to 800, per $m^2$ of the area.

The cross section per hole of the sieve tray is preferably 0.5 to 5 $cm^2$. The cross section per hole is more preferably 0.7 to 4 $cm^2$, further preferably 0.9 to 3 $cm^2$. The case is particularly preferred where the sieve tray has 100

to 1000 holes per $m^2$ of the area of the sieve portion, and the cross section per hole is 0.5 to 5 $cm^2$.

The step (II), when carried out, comprises: continuously supplying raw materials dialkyl carbonate and aryl alcohol (e.g., phenol) into a first continuous multistage distillation column containing a catalyst, simultaneously performing a reaction and distillation in the first continuous multistage distillation column, continuously discharging a first column low-boiling reaction mixture containing alcohols thus formed in a gas form from an upper portion of the first continuous multistage distillation column, and continuously discharging a first column high-boiling reaction mixture containing alkyl aryl carbonate (e.g., alkyl phenyl carbonate) thus formed in a liquid form from a lower portion of the first continuous multistage distillation column; and continuously supplying the first column high-boiling reaction mixture into a second continuous multistage distillation column containing a catalyst, simultaneously performing a reaction and distillation in the second continuous multistage distillation column, continuously discharging a second column low-boiling reaction mixture containing dialkyl carbonates thus formed in a gas form from an upper portion of the second continuous multistage distillation column, and continuously discharging a second column high-boiling reaction mixture containing diaryl carbonate (e.g., diphenyl carbonate) thus formed in a liquid form from a lower portion of the second continuous multistage distillation column to continuously produce diaryl carbonate (e.g., diphenyl carbonate).

The raw materials may contain reaction products alcohols, alkyl aryl carbonate (e.g., alkyl phenyl carbonate), diaryl carbonate (e.g., diphenyl carbonate), and alkyl aryl ether (e.g., alkyl phenyl ether) or reaction by-products such as high-boiling compounds, as described above. In the present embodiment to be actually industrially carried out, the raw materials preferably contain a small amount of these compounds, in consideration of equipment and cost required for separation and purification in other steps.

A column top component of the first continuous multistage distillation column in the step (II) often contains dialkyl carbonate, aryl alcohol (e.g., phenol), alkyl aryl ether, and the like in addition to alcohols formed through the reaction and may further contain a small amount of alkyl aryl carbonate, diaryl carbonate (e.g., diphenyl carbonate), and the like. This column top component of the first continuous multistage distillation column may be used directly as aliphatic monovalent alcohols in the step (I) and is preferably used as raw materials in the step (I) after the content of substances having a higher boiling point than that of the alcohols is decreased by distillation or the like. A mixture of the alcohols and dialkyl carbonate is particularly preferably used as aliphatic monovalent alcohols in the step (I).

In the step (II), the raw materials dialkyl carbonate and aryl alcohol (e.g., phenol), when continuously supplied into the first continuous multistage distillation column, may be supplied in a liquid form and/or in a gas form from an introduction port installed at one or several locations below an upper gas discharge port of the first distillation column and in an upper portion or an intermediate portion of the column, or a raw material rich in aryl alcohol (e.g., phenol) may be supplied in a liquid form from an introduction port in an upper portion of the first distillation column, while a raw material rich in dialkyl carbonate may be supplied in a gas form from an introduction port installed above a lower liquid discharge port of the first distillation column and in a lower portion of the column, and this is also a preferred method.

In the step (II), the first column high-boiling reaction mixture containing alkyl aryl carbonate (e.g., alkyl phenyl carbonate) continuously discharged from a lower portion of the first continuous multistage distillation column is continuously supplied into the second continuous multistage distillation column. As for a supply position thereof, the reaction mixture is preferably supplied in a liquid form and/or in a gas form from an introduction port installed at one or several locations below an upper gas discharge port of the second continuous multistage distillation column and in an upper portion or an intermediate portion of the column. In a preferred embodiment of the present embodiment, in the case of using a distillation column having a packing portion in an upper portion and a tray portion in a lower portion as the second continuous multistage distillation column, at least one introduction port is preferably installed between the packing portion and the tray portion. When the packing is formed from a plurality of (two or more) structured packings, an introduction port may be installed in space flanked by these structured packings, and this is also a preferred method.

In the step (II), reflux operation of condensing a gas component discharged from the column top of each of the first continuous multistage distillation column and the second continuous multistage distillation column, and then returning a portion of the condensate to the upper portion of each of the distillation columns is also a preferred method. In this case, the reflux ratio of the first continuous multistage distillation column is 0 to 10, and the reflux ratio of the second continuous multistage distillation column is in the range of 0.01 to 10, preferably 0.08 to 5, more preferably 0.1 to 2. In a preferred embodiment, the reflux ratio of the first continuous multistage distillation column is 0 which means that no reflux operation is performed.

In the step (II), the method for allowing the first continuous multistage distillation column to contain a homogeneous catalyst can be any method. The homogeneous catalyst is preferably supplied into the first continuous multistage distillation column from a position above the intermediate portion of the distillation column. Also, the catalyst is preferably supplied such that a reaction liquid comes into contact with the catalyst in a region of at least seven or more stages, preferably ten or more stages, more preferably 15 or more stages, of the first continuous multistage distillation column. In this case, a solution of the catalyst dissolved in raw materials or a reaction liquid may be introduced together with the raw materials, or this catalyst solution may be introduced from an introduction port different from that for the raw materials. In the present embodiment, the amount of the catalyst used in the first continuous multistage distillation column differs

depending on difference in the type of the catalyst used, the types and quantitative ratio of the raw materials, and reaction conditions such as a reaction temperature and a reaction pressure. The catalyst is used at usually 0.0001 to 30% by mass, preferably 0.005 to 10% by mass, more preferably 0.001 to 1% by mass, in terms of a ratio to the total mass of the raw materials.

In the step (II), the method for allowing the second continuous multistage distillation column to contain a homogeneous catalyst can be any method. The homogeneous catalyst is preferably supplied into the second continuous multistage distillation column from a position above the intermediate portion of the distillation column. In this case, a solution of the catalyst dissolved in raw materials or a reaction liquid may be introduced together with the raw materials, or this catalyst solution may be introduced from an introduction port different from that for the raw materials. In the present embodiment, the amount of the catalyst used in the second continuous multistage distillation column differs depending on difference in the type of the catalyst used, the types and quantitative ratio of the raw materials, and reaction conditions such as a reaction temperature and a reaction pressure. The catalyst is used at usually 0.0001 to 30% by mass, preferably 0.005 to 10% by mass, more preferably 0.001 to 1% by mass, in terms of a ratio to the total mass of the raw materials.

In the step (II), the catalyst for use in the first continuous multistage distillation column and the catalyst for use in the second continuous multistage distillation column may be of the same type or may be of different types. Preferably, the same type of catalyst is used. The catalyst is further preferably of the same type and can be dissolved in both reaction liquids. This embodiment is preferred because the catalyst is usually discharged, in a state dissolved in the high-boiling reaction mixture of the first continuous multistage distillation column, together with alkyl aryl carbonate (e.g., alkyl phenyl carbonate) and the like from a lower portion of the first distillation column, and supplied directly into the second continuous multistage distillation column. If necessary, the catalyst may be newly added to the second continuous multistage distillation column.

The reaction time of the transesterification reaction to be performed in the step (II) presumably corresponds to an average residence time of a reaction liquid in each of the first continuous multistage distillation column and the second continuous multistage distillation column. This reaction time differs depending on the shape and number of stages of the internal of each of the distillation columns, the amount of raw materials supplied, the type and amount of a catalyst, reaction conditions, etc. and is usually 0.01 to 10 hours, preferably 0.05 to 5 hours, more preferably 0.1 to 3 hours, in each of the first continuous multistage distillation column and the second continuous multistage distillation column.

The reaction temperature of the first continuous multistage distillation column differs depending on the type of the raw material compound used and the type and amount of a catalyst, and is usually in the range of 100 to 350°C. A higher reaction temperature is preferred for enhancing a reaction rate and however, is not preferred because such a high reaction temperature easily causes side reaction and increases by-products such as alkyl aryl ether (e.g., alkyl phenyl ether). In this respect, the reaction temperature of the first continuous multistage distillation column is in the range of preferably 130 to 280°C, more preferably 150 to 260°C, further preferably 180 to 250°C.

The reaction temperature of the second continuous multistage distillation column differs depending on the type of the raw material compound used and the type and amount of a catalyst, and is usually in the range of 100 to 350°C. A higher reaction temperature is preferred for enhancing a reaction rate and however, is not preferred because such a high reaction temperature easily causes side reaction and increases by-products such as alkyl aryl ether (e.g., alkyl phenyl ether), or Fries rearrangement reaction products of raw materials or the product alkyl aryl carbonate (e.g., alkyl phenyl carbonate) or diaryl carbonate (e.g., diphenyl carbonate) or their derivatives. In this respect, the reaction temperature of the second continuous multistage distillation column is in the range of preferably 130 to 280°C, more preferably 150 to 260°C, further preferably 180 to 250°C.

The reaction pressure of the first continuous multistage distillation column differs depending on the type and composition of the raw material compound used, the reaction temperature, etc., and can be any of reduced pressure, normal pressure, and increased pressure in the first continuous multistage distillation column. Usually, the column top pressure is in the range of 0.1 Pa to $2 \times 10^7$ Pa, preferably $10^5$ Pa to $10^7$ Pa, more preferably $2 \times 10^5$ Pa to $5 \times 10^6$ Pa.

The reaction pressure of the second continuous multistage distillation column differs depending on the type and composition of the raw material compound used, the reaction temperature, etc., and can be any of reduced pressure, normal pressure, and increased pressure. Usually, the column top pressure is in the range of 0.1 Pa to $2 \times 10^7$ Pa, preferably $10^3$ Pa to $10^6$ Pa, more preferably $5 \times 10^3$ Pa to $10^5$ Pa.

Two or more distillation columns may be used as the first continuous multistage distillation column in the step (II). In this case, the two or more distillation columns may be connected in series, may be connected in parallel, or may be connected with in series and in parallel in combination. Two or more distillation columns may be used as the second continuous multistage distillation column in the step (II). In this case, the two or more distillation columns may be connected in series, may be connected in parallel, or may be connected with in series and in parallel in combination.

The material constituting the first continuous multistage distillation column and the second continuous multistage distillation column for use in the step (II) is mainly a metal material such as carbon steel or stainless steel, and stainless steel is preferred from the viewpoint of the quality of the produced aromatic carbonate.

A material that is composed mainly of Fe and contains 2% by mass or more of Mo and 18% by mass or less of Cr is preferably used in a wetted portion in a lower portion of the second continuous multistage distillation column. Use of such a material in a wetted portion in a lower portion of the second continuous multistage distillation column tends to be able to suppress the corrosion of the distillation column even if the concentration of a high-boiling substance in a component is elevated in the lower portion of the second continuous multistage distillation column.
In the present embodiment, the term "composed mainly of" means 50% by mass or more.

Examples

Hereinafter, the present invention will be described further specifically with reference to Examples. However, the present invention is not limited by the following Examples.

[Analysis method]

The analysis of each composition such as the concentrations of diphenyl carbonate (DPC), a high-boiling substance (HB), and a catalyst in a column bottom component, the concentration of alkyl aryl carbonate in a low-boiling reaction mixture, and the purity or impurity component content of diphenyl carbonate was conducted by gas chromatography. The analysis by gas chromatography was conducted by the internal standard method, and the internal standard substance used was toluene.

Apparatus: 7890A manufactured by Agilent Technologies, Inc.
Column: DB-1 (Length 30 m × I.D. 0.25 mm × Film 0.25 μm) manufactured by Agilent J&W
Carrier gas: He at a flow rate of 2.0 mL/min
Injection port temperature: 250°C
Injection volume: 1.0 μE
Oven temperature: 50°C kept for 2 minutes, followed by heating at a heating rate of 10°C/min to 300°C, which was kept for 8 minutes.
Injection method: split method with a split ratio of 20 Detector: FID with a temperature of 300°C

0.1 g of a sampled column bottom component (Ab) was weighed, and 0.04 g of toluene (precisely weighed on a 0.1-mg basis in all cases) and 5 mL of acetonitrile were added thereto to prepare a sample solution for gas chromatography. The prepared solution was filtered through a 0.45 to 0.50 μm polytetrafluoroethylene (PTFE) membrane filter. The relationship between the peak area ratio and concentration ratio of DPC and the internal standard (toluene) was determined by measuring in advance each of DPC and toluene having known concentrations, and the gas chromatography measurement value of the column bottom component was corrected. A remaining amount [% by mass] determined by subtracting the DPC concentration [% by mass] obtained by gas chromatography measurement from 100 [% by mass] is the amount of the high-boiling substance (HB) and the catalyst.
In catalyst concentration measurement, organic materials of the column bottom component (Ab) were degraded with microwave. Then, the metal concentration [% by mass] of the catalyst was measured using an ICP-optical emission spectrometer (ICP). The amount of the catalyst [% by mass] was determined by calculation from the molecular weight of the catalyst.

<Example 1>

A mixture containing diaryl carbonate (diphenyl carbonate) was produced as follows by use of the same apparatus and production method as in Example 1 of International Publication No. WO 2007/69529 except that a catalyst used in <reactive distillation> of (2) a step (II) of continuously producing diphenyl carbonate was changed to a catalyst described in Example 1 of International Publication No. WO 2011/105442.

(Preparation of titanium-containing composition)

A 60 L batch reactor equipped with a stirrer, a heater, and a distillation column was charged with 7 kg of tetrabutoxytitanium (manufactured by DuPont de Nemours, Inc., product name: Tyzor TnBT) in a nitrogen atmosphere and subsequently charged with 14 kg of phenol purified by distillation in advance.
Next, the mixture in the batch reactor was heated to 180°C by the heater at ordinary temperature and reacted. n-Butanol generated through the reaction was recovered from the column top of the distillation column. Further, the pressure of the batch reactor was reduced to approximately 53 kPa, and n-butanol was recovered.
Then, the batch reactor was returned to ordinary pressure and charged with approximately 18 kg of diphenyl

carbonate, and the mixture in the reactor was heated to approximately 190°C. Next, the pressure of the batch reactor was reduced to approximately 1.3 kPa, and diphenyl carbonate containing a low-boiling component was distilled off to obtain a titanium-containing composition. Diphenyl carbonate was added to the obtained titanium-containing composition such that its titanium concentration was 5% by mass. The titanium-containing composition was heated at 200°C for approximately 48 hr and then used as a reaction catalyst in subsequent production of diphenyl carbonate.

<First continuous multistage distillation column 101>

The first continuous multistage distillation column used was a continuous multistage distillation column with $L_1$ = 3300 cm, $D_1$ = 500 cm, $L_1/D_1$ = 6.6, $n_1$ = 80, $D_1/d_{11}$ = 17, and $D_1/d_{12}$ = 9 as shown in Figure 4. In this Example, the internal used was a sieve tray with cross section per hole = approximately 1.5 $cm^2$ and the number of holes = approximately 250 holes/$m^2$.

<Second continuous multistage distillation column 201>

The second continuous multistage distillation column used was a continuous multistage distillation column with $L_2$ = 3100 cm, $D_2$ = 500 cm, $L_2/D_2$ = 6.2, $n_2$ = 30, $D_2/d_{21}$ = 3.85, and $D_2/d_{22}$ = 11.1 as shown in Figure 5. In this Example, the internal used was two packings of Mellapak (total theoretical number of stages: 11) installed in an upper portion and a sieve tray with cross section per hole = approximately 1.3 $cm^2$ and the number of holes = approximately 250 holes/$m^2$ in a lower portion. A material containing 2.5% by mass of a Mo component and 17% by mass of a Cr component was used in a wetted portion of a lower portion. A test piece 1 containing no Mo component and containing 19% by mass of the Cr component, and a test piece 2 containing 0.15% by mass of the Mo component and 0.4% by mass of the Cr component were placed as test pieces in the wetted portion of the lower portion.

<Production of diphenyl carbonate>

An apparatus comprising the first continuous multistage distillation column 101 and the second continuous multistage distillation column 201 connected with each other, as shown in Figure 6, was used to produce diphenyl carbonate.

A raw material 1 consisting of phenol/dimethyl carbonate = 1.9 (mass ratio) was continuously introduced in a liquid form at a flow rate of 57 ton/hr from an upper introduction port 11 of the first continuous multistage distillation column 101. Meanwhile, a raw material 2 consisting of dimethyl carbonate/phenol = 3.6 (mass ratio) was continuously introduced in a gas form at a flow rate of 57 ton/hr from a lower introduction port 12 of the first continuous multistage distillation column 101. The molar ratio of the raw materials introduced to the first continuous multistage distillation column 101 was dimethyl carbonate/phenol = 1.35. The titanium-containing composition prepared as described above was continuously introduced as a catalyst at a flow rate of 188 kg/hr from a bottom portion 11 of a purifying column A to the upper introduction port 11 of the first continuous multistage distillation column 101 such that the concentration was approximately 100 ppm in the liquid of the first continuous multistage distillation column 101. In the first continuous multistage distillation column 101, reactive distillation was continuously performed under conditions involving a column bottom temperature of 225°C, a column top pressure of $7 \times 10^5$ Pa, and a reflux ratio of 0. A first column low-boiling reaction mixture of the first continuous multistage distillation column 101 containing methyl alcohol, dimethyl carbonate, phenol, and the like was continuously discharged in a gas form from a column top portion 13 and discharged at a flow rate of 39 ton/hr from a discharge port 16 through a heat exchanger 14. Meanwhile, a first column high-boiling reaction mixture of the first continuous multistage distillation column 101 containing methyl phenyl carbonate, dimethyl carbonate, phenol, diphenyl carbonate, the catalyst, and the like was continuously discharged in a liquid form from an introduction port 21 through a column bottom portion 17.

Because of reaching a stable steady state 24 hours later, the first column high-boiling reaction mixture from the column bottom portion 17 of the first continuous multistage distillation column 101 was continuously supplied directly at a flow rate of 75 ton/hr from the raw material introduction port 21 installed between the Mellapak and the sieve tray of the second continuous multistage distillation column 201. The second continuous multistage distillation column 201 was allowed to contain the titanium-containing composition prepared as described above as a catalyst, and reactive distillation was continuously performed under conditions involving a column bottom temperature of 210°C, a column top pressure of $3 \times 10^4$ Pa, and a reflux ratio of 0.3. Stable steady operation was able to be achieved 24 hours later. A second column low-boiling reaction mixture containing 35% by mass of dimethyl carbonate, 56% by mass of phenol, and 0.5% by mass of methyl phenyl carbonate was continuously discharged from a column top portion 23 of the second continuous multistage distillation column 201, and the flow rate at a discharge port 26 was 62 ton/hr. A second column high-boiling reaction mixture containing 38% by mass of methyl phenyl carbonate and 56% by mass of diphenyl carbonate was continuously discharged from a column bottom portion 27. The second column low-boiling reaction mixture of the

second continuous multistage distillation column 201 was continuously supplied into the first continuous multistage distillation column 101 from the introduction port 11 (first circulation step). In this respect, the amounts of dimethyl carbonate and phenol newly supplied were adjusted such that the composition and amounts of the raw material 1 and the raw material 2 were maintained, in light of the composition and the amount of the second column low-boiling reaction mixture.

<Purification of diphenyl carbonate>

Figure 1 is a schematic view of a process flow of a method for purifying diphenyl carbonate used in Example 1. In this Figure 1, the left diagram shows a purifying column A, and the right diagram shows a diaryl carbonate (diphenyl carbonate) purifying column B. In Figure 1, reference numeral 14 denotes a column top heat exchanger (condenser) of the purifying column A, and reference numeral 18 denotes a heat exchanger (reboiler) of the purifying column A. Likewise, reference numeral 24 denotes a column top heat exchanger (condenser) of the purifying column B, and reference numeral 28 denotes a heat exchanger (reboiler) of the purifying column B. The type of the reboiler of the purifying column A was forced circulation type, and the type of the reboiler of the purifying column B was thermosyphon type. The packings of the purifying column A and the purifying column B were structured packings. The structured packing of the purifying column A was Mellapak (manufactured by Sulzer Ltd.), and the structured packing of the purifying column B was Mellapak (manufactured by Sulzer Ltd.).

In Example 1, the purifying column A employed stainless steel as a material and had the theoretical number of stages of 19 and a side cut discharge port $A_2$. In the purifying column A, the side cut discharge port $A_2$ was positioned at the 4th stage counted from above in terms of the theoretical stage. In the purifying column A, crude ethylene carbonate was introduced from a column bottom portion $A_1$. The purifying column B employed stainless steel as a material and had the theoretical number of stages of 20 and a side cut discharge port $B_2$. In the purifying column B, a column bottom component (Ab) of the purifying column A was introduced from a position of the 5th stage counted from above in terms of the theoretical stage. In the purifying column B, the side cut discharge port $B_2$ was positioned at the 18th stage counted from above in terms of the theoretical stage.

A liquid was continuously introduced at 26300 kg/hr to the introduction port $A_1$ of the purifying column A. The introduction liquid (second column high-boiling reaction mixture) to be continuously introduced to the purifying column A had a diphenyl carbonate (DPC) content of 56% by mass, a methyl phenyl carbonate (MPC) content of 26% by mass, a high-boiling component content of 13% by mass, and a catalytic component content of 1% by mass.

The discharge flow rate of a low-boiling component (column top component (At)) from a column top 16 of the purifying column A was 6800 kg/hr. A circulation rate of a catalytic component (column bottom component (Ab)) from a column bottom 17 of the purifying column A was 5380 kg/hr. A side cut component (As) containing diphenyl carbonate was discharged at a ratio of 14100 kg/hr from the side cut discharge port $A_2$ of the purifying column A and introduced to the purifying column B. In the purifying column B, the discharge flow rate of a low-boiling component (column top component (Bt)) from a column top 26 was 1200 kg/hr, and the discharge flow rate of a high-boiling component (column bottom component (Bb)) from a column bottom 27 was 900 kg/hr. In the purifying column B, diphenyl carbonate was discharged as a side cut component (Bs) at a ratio of 12000 kg/hr (production amount (BsF) = 12 t/hr) from the side cut discharge port $B_2$. In the purifying column B, the diphenyl carbonate discharged from the side cut discharge port $B_2$ had a high-boiling component (hereinafter, also referred to as "HB") concentration of 7 ppm by mass and an altered substance (XA) concentration of 10 ppm by mass. The diphenyl carbonate obtained from the side cut discharge port $B_2$ of the diphenyl carbonate purifying column B had a purity of 99.99% or more.

The operation conditions of the purifying column A involved a reflux ratio of 3.49, a column top reflux rate of 23700 kg/hr, and a column bottom temperature of 206°C. The amount of vapor used in the reboiler of the purifying column A was 5300 kg/hr.

The operation conditions of the purifying column B involved a reflux ratio of 1.87, a column top reflux rate of 2250 kg/hr, and a column bottom temperature of 211°C. The amount of vapor used in the reboiler of the purifying column B was 3600 kg/hr.

The operation results of Example 1 are shown in Table 1.

<Examples 2 to 7>

Diphenyl carbonate was produced in the same manner as in Example 1 except that the conditions of the purifying columns A and B were changed as shown in Table 1. The operation results of each Example are shown in Table 1.

<Comparative Example 1>

Figure 2 is a schematic view of a process flow of a method for purifying diphenyl carbonate used in Comparative

Example 1.

Diphenyl carbonate was produced in the same manner as in Example 1 except that as shown in Figure 2, the purifying column A used was a continuous multistage distillation column having no side cut discharge port; and the conditions of the purifying columns A and B were changed as shown in Table 1. The operation results of Comparative Example 1 are shown in Table 1. As shown in Table 1, in Comparative Example 1, the ratio of the amount of discharge from the column bottom to the amount of supply (the amount of discharge from the column bottom / the amount of supply) in the purifying column A was large; thus, the amount of loss in purification was large as compared with Examples. In Comparative Example 1, the amount of vapor used in the reboilers of the purifying columns A and B was large with respect to a production amount; thus, consumed energy in purification was large as compared with Examples. Particularly, in Comparative Example 1, the amount of vapor used in the reboiler of the purifying column B was very large with respect to a production amount; thus, consumed energy in the purifying column B was markedly large as compared with Examples.

Table 1 shows respective conditions and results of Examples 1 to 7 and Comparative Example 1.

[Table 1]

| | | Unit | Example 1 | Example 2 | Example 3 | Example 4 | Example 5 | Example 6 | Example 7 | Comparative Example 1 |
|---|---|---|---|---|---|---|---|---|---|---|
| Conditions, etc. for purifying column A | Supplied composition | % by mass | DPC: 56, MPC: 26. high-boiling component: 13, catalytic component: 1 | | | | | | | |
| | Amount of supply | kg/hr | 26300 | 26300 | 26300 | 1500 | 70000 | 26300 | 26300 | 26300 |
| | Discharge from column top of column A | kg/hr | 6800 | 6800 | 4800 | 388 | 18100 | 6800 | 6800 | 20885 |
| | Catalyst circulation in column A | kg/hr | 5380 | 5380 | 5380 | 307 | 14300 | 5380 | 5380 | 5380 |
| | Discharge from column bottom of column A | kg/hr | 20 | 20 | 26 | 1 | 50 | 20 | 30 | 35 |
| | Side cut discharge of column A | kg/hr | 14100 | 14100 | 16094 | 804 | 37550 | 14100 | 14090 | - |
| | DPC concentration in side cut discharged liquid of column A | % by mass | 99.0 | 99.0 | 86.9 | 99.0 | 99.0 | 99.0 | 99.0 | 62.4 |
| | Reflux ratio of column A | - | 3.49 | 6.24 | 4.69 | 3.49 | 3.49 | 11.21 | 2.82 | 0.55 |
| | Amount of reflux of column A | kg/hr | 23700 | 42400 | 22500 | 1355 | 63200 | 53300 | 19200 | 11500 |
| | Amount of vapor of column A | kg/hr | 5300 | 8500 | 4800 | 300 | 14100 | 10500 | 4500 | 5300 |
| | Total theoretical stage of column A | Plate number | 19 | 13 | 24 | 19 | 19 | 12 | 25 | 25 |
| | Theoretical stage in lower portion of column A (An) | Plate number | 16 | 10 | 19 | 16 | 16 | 9 | 22 | |
| | Column diameter of column A | m | 4.6 | 5.8 | 4.3 | 1.1 | 7.5 | 6.4 | 4.3 | 3.4 |
| | Cross section of column A (SA) | $m^2$ | 16.6 | 26.4 | 14.5 | 0.9 | 44.2 | 32.2 | 14.5 | 9.1 |
| | Column bottom temperature of column A | °C | 206 | 197 | 210 | 206 | 206 | 196 | 211 | 211 |

(continued)

| | | Unit | Example 1 | Example 2 | Example 3 | Example 4 | Example 5 | Example 6 | Example 7 | Comparative Example 1 |
|---|---|---|---|---|---|---|---|---|---|---|
| Conditions, etc. for purifying column B | Discharge from column top of column B | kg/hr | 1200 | 1200 | 3200 | 68 | 3200 | 1200 | 1200 | 8000 |
| | Discharge from column bottom of column B | kg/hr | 900 | 900 | 900 | 51 | 2400 | 900 | 900 | 900 |
| | Yield (BsF) | kq/hr | 12000 | 12000 | 11994 | 685 | 31950 | 12000 | 11990 | 11985 |
| | XA concentration in product | ppm by mass | 10 | 15 | 9 | 10 | 10 | 30 | 10 | 20 |
| | Reflux ratio of column B | - | 1.88 | 2.63 | 2.63 | 1.87 | 1.87 | 1.87 | 1.87 | 1.7 |
| | Amount of reflux of column B | kg/hr | 2250 | 3150 | 8400 | 127 | 5990 | 2240 | 2240 | 13600 |
| | Amount of vapor of column B | kg/hr | 3600 | 3800 | 6000 | 200 | 9600 | 3600 | 3600 | 9900 |
| | Upper column diameter of column B (DB1) | m | 1.6 | 2.9 | 3.2 | 0.4 | 2.6 | 1.6 | 1.6 | 2.8 |
| | Lower column diameter of column B (DB2) | m | 2.8 | 3.8 | 3.8 | 0.7 | 4.6 | 2.8 | 2.8 | |
| | Column bottom temperature of column B | °C | 211 | 211 | 211 | 211 | 211 | 211 | 211 | 211 |
| Operation results | Amount of discharge from column bottom of column A/ amount of supply | kg/ton | 0.76046 | 0.76046 | 0.98859 | 0.66667 | 0.71429 | 0.76046 | 1.14068 | 1.3308 |
| | Amount of vapor of column B/production amount | - | 0.300 | 0.317 | 0.500 | 0.292 | 0.300 | 0.300 | 0.300 | 0.826 |
| | Amount of vapor of columns A + B / production amount | - | 0.742 | 1.025 | 0.900 | 0.730 | 0.742 | 1.175 | 0.676 | 1.268 |
| | SA/BsF | - | 1.4 | 2.2 | 1.2 | 1.4 | 1.4 | 2.7 | 1.2 | 0.8 |
| | DB1/DB2 | - | 0.6 | 0.8 | 0.8 | 0.6 | 0.6 | 0.6 | 0.6 | 1.0 |

The present application is based on Japanese Patent Application No. 2021-163793 filed on October 5, 2021, the contents of which are incorporated herein by reference.

Industrial Applicability

By carrying out the method of the present invention, for example, high-purity diaryl carbonate necessary for producing high-quality and high-performance aromatic polycarbonate can be produced at an industrial scale of 0.5 tons or more, preferably 1 ton or more, more preferably 2 tons or more, further preferably 3 tons or more, per hour in a state where the amount of loss is small and an energy consumption can be suppressed. Thus, the method of the present invention is excellently effective as a method for industrially producing high-purity diaryl carbonate.

Reference Signs List

(Figures 1 and 2)

$A_1$: crude ethylene carbonate introduction port
$A_2$: side cut discharge port of purifying column A
$B_1$: introduction port of purifying column B
$B_2$: side cut discharge port of purifying column B
13 and 23: column top gas discharge port
18 and 28: heat exchanger (reboiler)
14 and 24: heat exchanger (condenser)
16 and 26: column top component discharge port
17 and 27: column bottom liquid discharge port
11, 12, and 31: column bottom component discharge port
15 and 25: reflux liquid introduction port
$D_A$: column diameter of purifying column A
$D_B$, $D_{B1}$, $D_{B2}$: column diameter of purifying column B

(Figure 3)

1: gas discharge port
2: liquid discharge port
3-a to 3-e: introduction port
4-a to 4-b: introduction port
5: head plate portion
6: internal
7: body portion
10: continuous multistage distillation column
$L_0$: body length (cm)
$D_0$: body inside diameter (cm)
$d_{01}$: inside diameter (cm) of gas discharge port
$d_{02}$: inside diameter (cm) of liquid discharge port

(Figures 4, 5, and 6)

1: gas discharge port
2: liquid discharge port
3: introduction port
4: introduction port
5: head plate portion
$L_1$ and $L_2$: body length (cm)

$D_1$ and $D_2$: body inside diameter (cm)
$d_{11}$ and $d_{21}$: gas discharge port inside diameter (cm)
$d_{12}$ and $d_{22}$: liquid discharge port inside diameter (cm)
101: first continuous multistage distillation column
201: second continuous multistage distillation column
11, 12, and 21: introduction port
13 and 23: column top gas discharge port
14, 24, 18, and 28: heat exchanger
17 and 27: column bottom liquid discharge port
16 and 26: column top component discharge port
31: column bottom component discharge port of second continuous multistage distillation column

**Claims**

**1.** A method for continuously producing high-purity diaryl carbonate, the method for industrially producing high-purity diaryl carbonate and comprising:

a first purification step of continuously introducing a mixture containing diaryl carbonate to a purifying column A having a side cut discharge port, and continuously separating by distillation the mixture into a column top component (At), a side cut component (As) containing diaryl carbonate, and a column bottom component (Ab) containing a catalyst; and
a second purification step of continuously introducing the side cut component (As) to a diaryl carbonate purifying column B having a side cut discharge port, and continuously separating by distillation the component into three components, a column top component (Bt), a side cut component (Bs), and a column bottom component (Bb), thereby obtaining high-purity diaryl carbonate as the side cut component (Bs).

**2.** The production method according to claim 1, wherein the mixture contains the diaryl carbonate at a ratio of 40% by mass or more and 90% by mass or less.

**3.** The production method according to claim 1, wherein the mixture contains alkyl aryl carbonate at a ratio of 10% by mass or more and 50% by mass or less.

**4.** The production method according to claim 1, wherein a content of the diaryl carbonate in the side cut component (As) is 80% by mass or more, and a content of the diaryl carbonate in the side cut component (Bs) is 99.1% by mass or more.

**5.** The production method according to any one of claims 1 to 3, wherein the theoretical number of stages An from the column bottom to the side cut discharge port of the purifying column A is in a range of 10 or more and 19 or less.

**6.** The production method according to claim 1, wherein a ratio (SA/BsF) of column cross section (SA ($m^2$)) of the purifying column A to a production amount (BsF (ton/hr)) as the discharged side cut component (Bs) is in a range of 0.5 or more and 5 or less.

**7.** The production method according to claim 1, wherein a ratio (DB1/DB2) of an upper column diameter (DB1 (m)) to a lower column diameter (DB2 (m)) in the purifying column B is in a range of 0.1 or more and 0.9 or less.

**8.** The production method according to claim 4, wherein the ratio (DB1/DB2) of an upper column diameter (DB1 (m)) to a lower column diameter (DB2 (m)) in the purifying column B is in a range of 0.1 or more and 0.9 or less.

**9.** The production method according to claim 1, wherein the purifying column A comprises at least one reboiler selected from the group consisting of forced circulation type, cross pipe falling film type, and thin-film evaporation type.

**10.** The production method according to claim 1, wherein

a packing of the purifying column A and/or the purifying column B is a structured packing, wherein
the structured packing comprises at least one selected from the group consisting of Mellapak, Gempak, Techno Pack, Flexipac, Sulzer Packings, Goodloe Packing, Glitsch Grid, and a gauze packing.

**11.** The production method according to claim 1, wherein the mixture is a reaction mixture obtained through at least one reaction selected from the group consisting of (i) transesterification reaction of dialkyl carbonate and aryl alcohol, (ii) disproportionation reaction of alkyl aryl carbonate, and (iii) transesterification reaction of alkyl aryl carbonate and aryl alcohol, in the presence of a catalyst.

**12.** The production method according to claim 1, further comprising the steps of:

continuously supplying dialkyl carbonate and aryl alcohol into a first continuous multistage distillation column containing a catalyst, simultaneously performing a reaction and distillation in the first continuous multistage distillation column, continuously discharging a first column low-boiling reaction mixture containing alcohols thus formed in a gas form from an upper portion of the first continuous multistage distillation column, and continuously discharging a first column high-boiling reaction mixture containing alkyl aryl carbonate thus formed in a liquid form from a lower portion of the first continuous multistage distillation column to continuously produce alkyl aryl carbonate; and

continuously supplying the first column high-boiling reaction mixture into a second continuous multistage distillation column containing a catalyst, simultaneously performing a reaction and distillation in the second continuous multistage distillation column, continuously discharging a second column low-boiling reaction mixture containing dialkyl carbonates thus formed in a gas form from an upper portion of the second continuous multistage distillation column, and continuously discharging a second column high-boiling reaction mixture containing diaryl carbonate thus formed in a liquid form from a lower portion of the second continuous multistage distillation column to continuously produce diaryl carbonate, wherein

the mixture is the second column high-boiling reaction mixture containing diaryl carbonate.

**13.** The production method according to claim 1, wherein the diaryl carbonate is diphenyl carbonate.

[Figure 1]

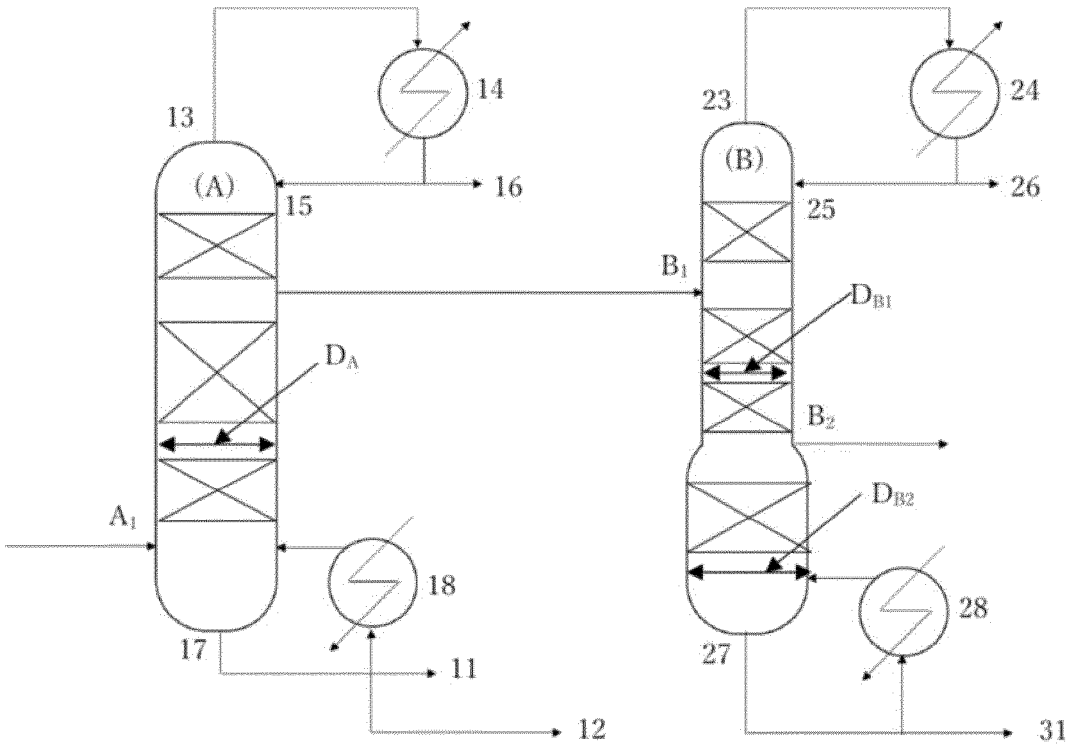

[Figure 2]

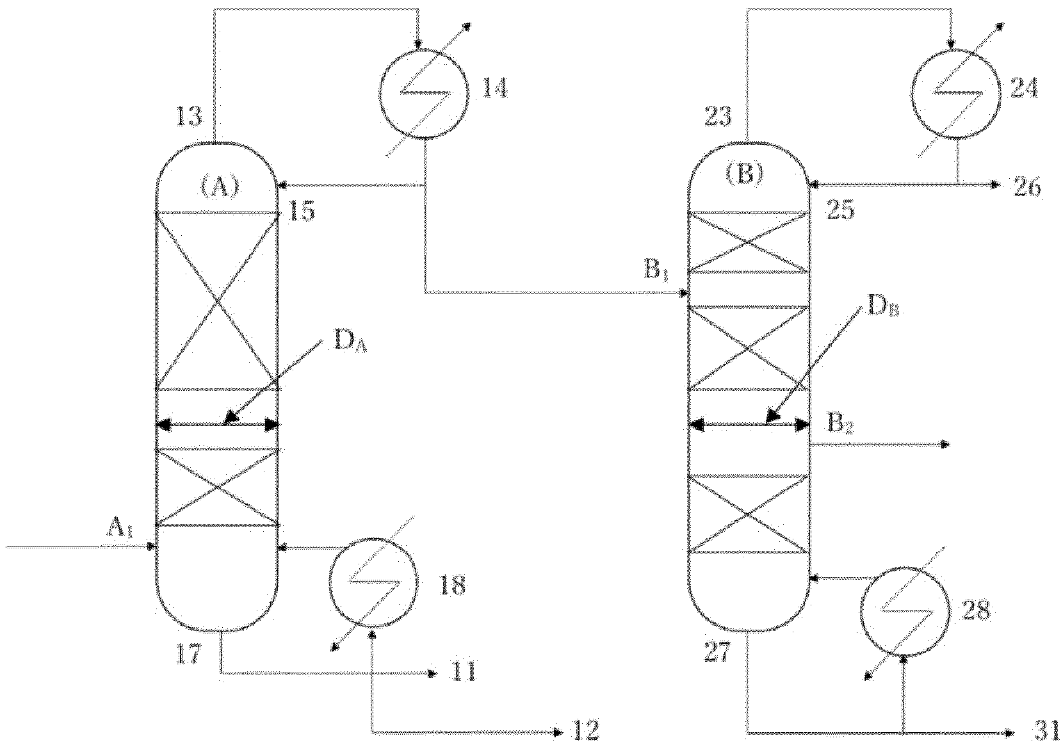

[Figure 3]

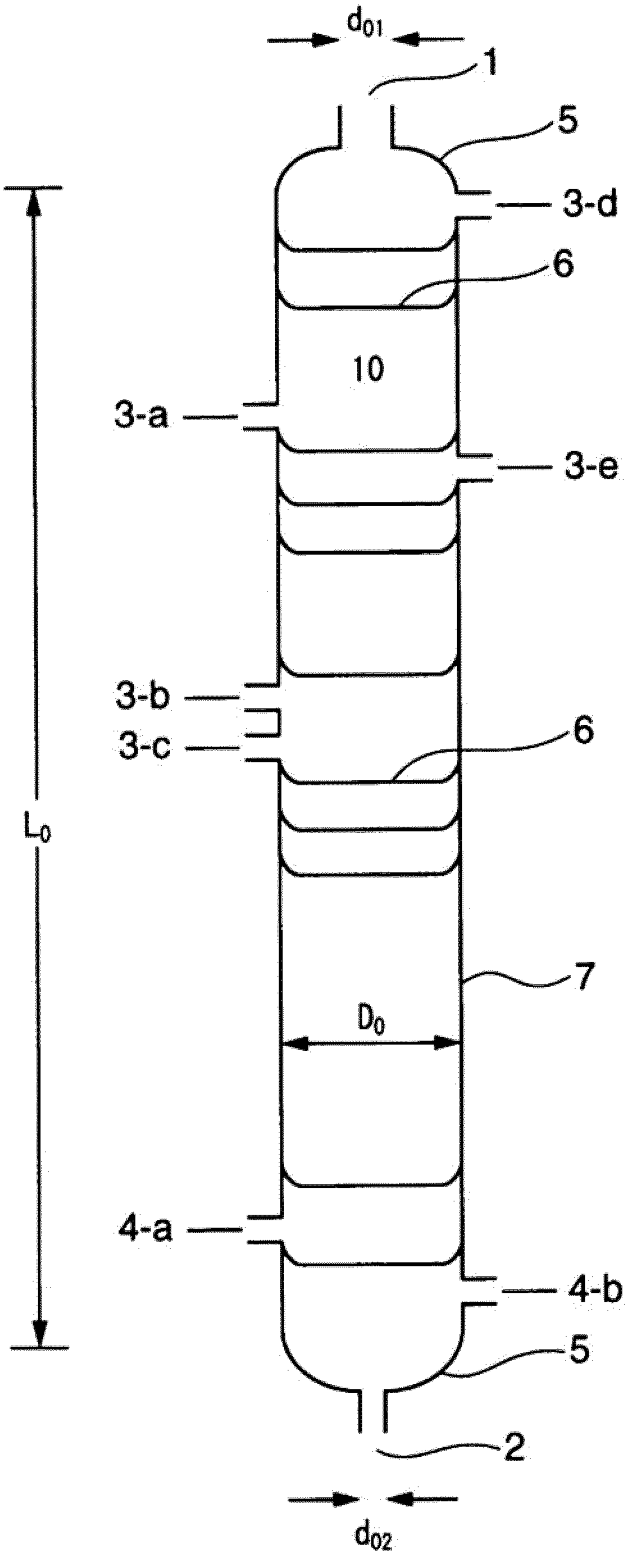

[Figure 4]

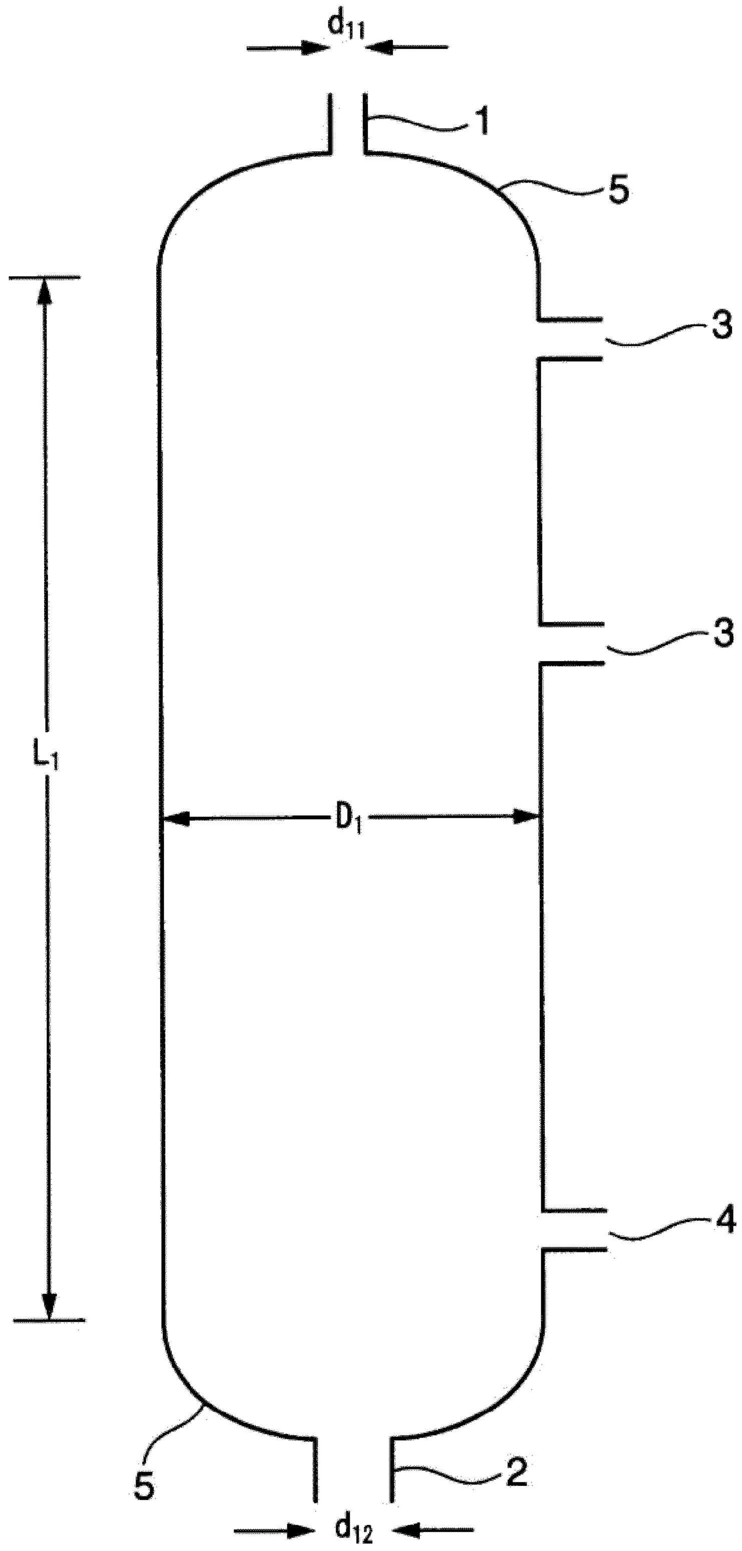

[Figure 5]

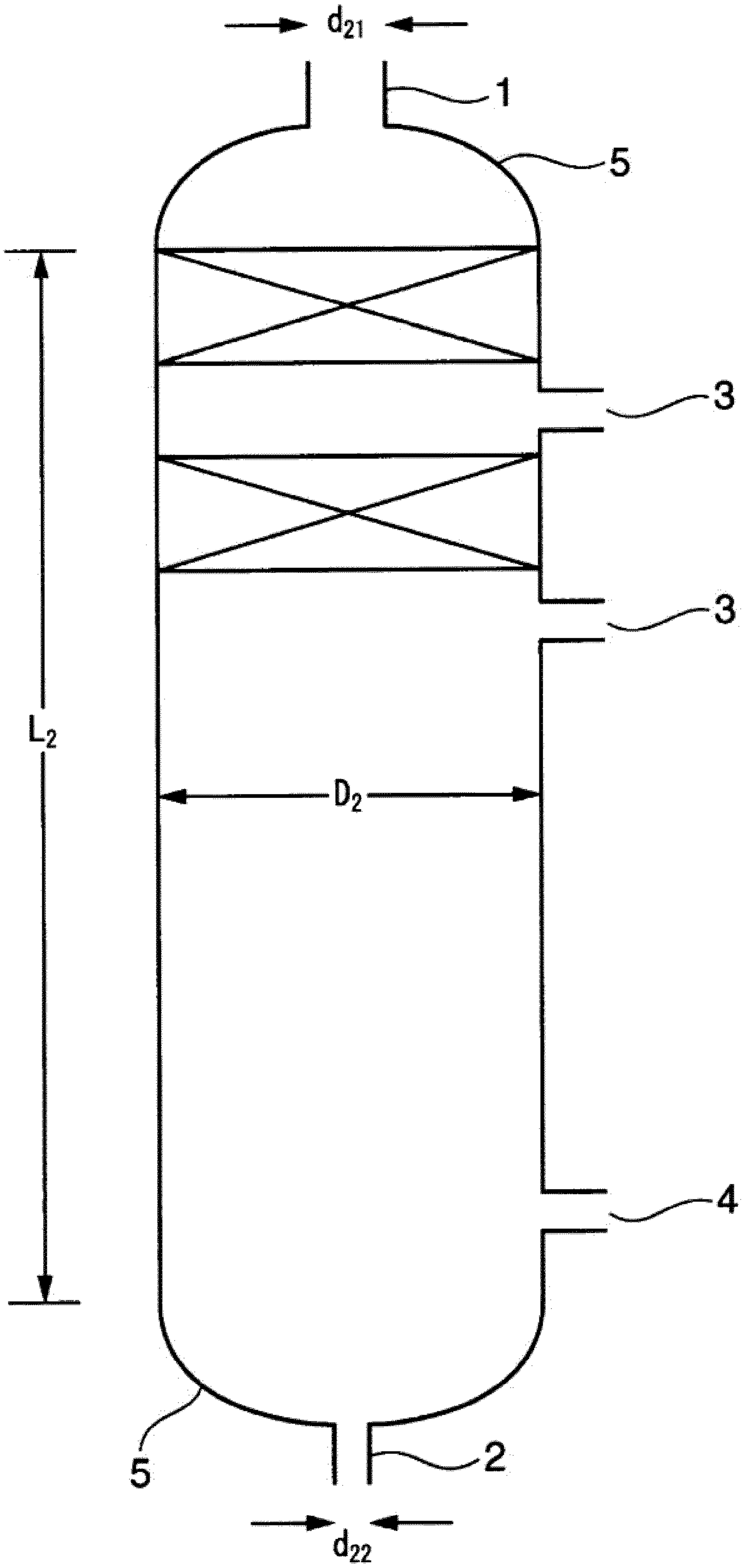

[Figure 6]

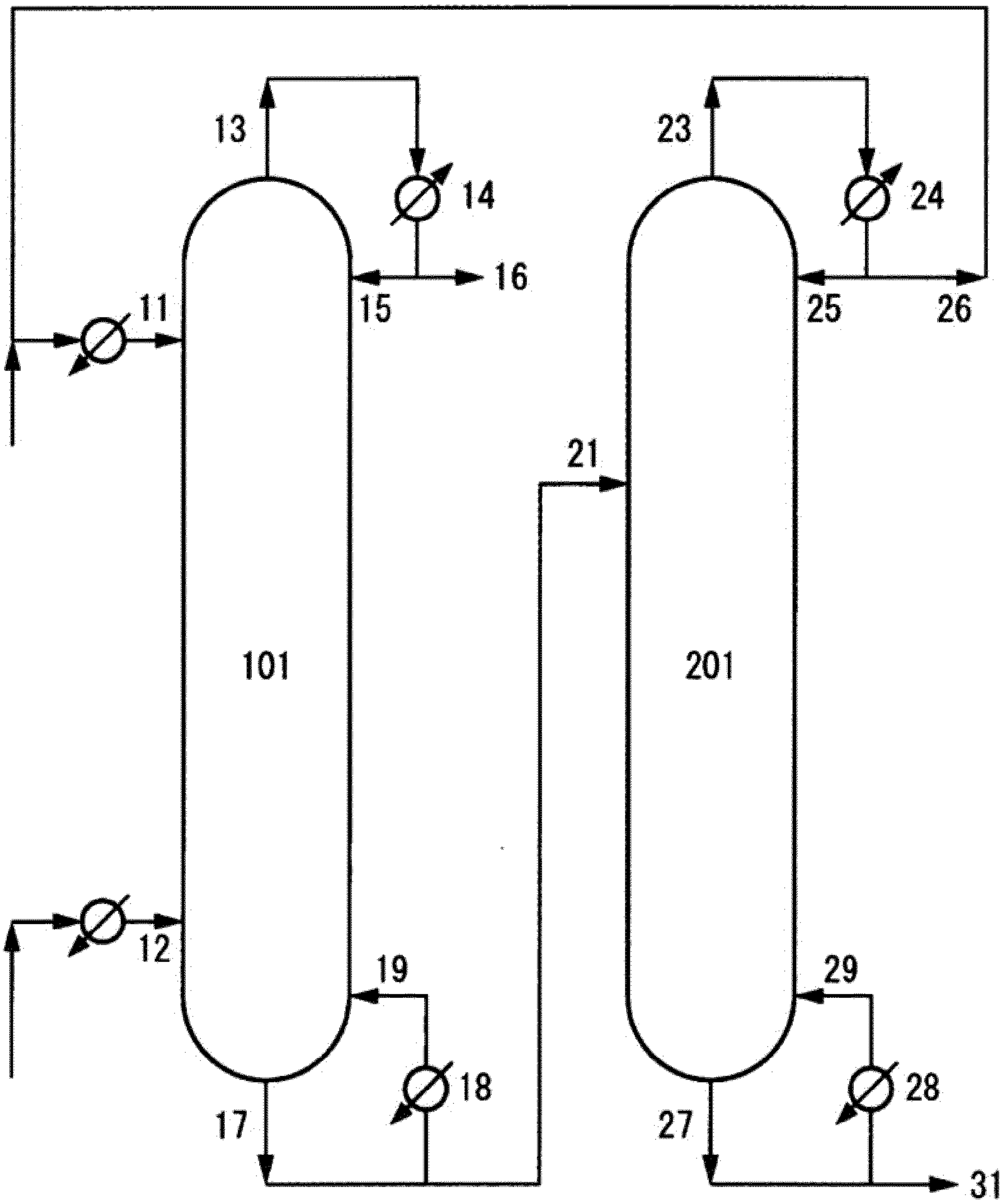

## INTERNATIONAL SEARCH REPORT

International application No.
**PCT/JP2022/037264**

**A. CLASSIFICATION OF SUBJECT MATTER**

***C07C 68/08***(2006.01)i; ***B01D 3/14***(2006.01)i; ***C07C 68/06***(2020.01)i; ***C07C 69/96***(2006.01)i
FI: C07C68/08; C07C69/96 Z; C07C68/06; B01D3/14 A

According to International Patent Classification (IPC) or to both national classification and IPC

**B. FIELDS SEARCHED**

Minimum documentation searched (classification system followed by classification symbols)

C07C68/08; B01D3/14; C07C68/06; C07C69/96

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2009-137952 A (BAYER MATERIALSCIENCE AG) 25 June 2009 (2009-06-25) claims 1, 3, paragraphs [0006], [0035], [0038], [0042], [0047], [0048], fig. 1.1 | 1-5, 9-13 |
| Y | CN 107400055 A (TIANJIN UNIVERSITY OF SCIENCE & TECHNOLOGY) 28 November 2017 (2017-11-28) claim 1, paragraph [0016], fig. 1 | 1-5, 9-13 |
| Y | JP 58-099433 A (NIPPON SHOKUBAI KAGAKU KOGYO CO., LTD.) 13 June 1983 (1983-06-13) p. 3, column 4 | 9 |
| Y | JP 2002-053657 A (UBE INDUSTRIES, LTD.) 19 February 2002 (2002-02-19) paragraph [0077] | 10 |
| Y | JP 09-301931 A (MITSUBISHI CHEMICAL CORP.) 25 November 1997 (1997-11-25) paragraph [0014] | 10 |
| Y | WO 2011/105442 A1 (ASAHI KASEI CHEMICALS CORP.) 01 September 2011 (2011-09-01) claim 1, example 1, fig. 1 | 12 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 November 2022** | **06 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/037264**

**C. DOCUMENTS CONSIDERED TO BE RELEVANT**

| Category* | Citation of document, with indication, where appropriate, of the relevant passages | Relevant to claim No. |
|---|---|---|
| A | WO 2007/063757 A1 (ASAHI KASEI CHEMICALS CORP.) 07 June 2007 (2007-06-07) entire text, all drawings | 1-13 |
| A | WO 2007/069529 A1 (ASAHI KASEI CHEMICALS CORP.) 21 June 2007 (2007-06-21) entire text, all drawings | 1-13 |
| A | WO 2006/043491 A1 (ASAHI KASEI CHEMICALS CORP.) 27 April 2006 (2006-04-27) entire text, all drawings | 1-13 |

**INTERNATIONAL SEARCH REPORT**
**Information on patent family members**

International application No.
**PCT/JP2022/037264**

| Patent document cited in search report | Publication date (day/month/year) | Patent family member(s) | Publication date (day/month/year) |
|---|---|---|---|
| JP 2009-137952 A | 25 June 2009 | US 2009/0137832 A1<br>claims 1, 3, paragraphs [0006], [0049], [0052], [0056], [0111], [0112], fig. 1.1<br>EP 2062868 A2 | |
| CN 107400055 A | 28 November 2017 | (Family: none) | |
| JP 58-099433 A | 13 June 1983 | (Family: none) | |
| JP 2002-053657 A | 19 February 2002 | US 2002/0019512 A1<br>paragraph [0094]<br>EP 1178033 A1 | |
| JP 09-301931 A | 25 November 1997 | (Family: none) | |
| WO 2011/105442 A1 | 01 September 2011 | US 2012/0316357 A1<br>claim 1, example 1, fig. 1<br>EP 2540697 A1 | |
| WO 2007/063757 A1 | 07 June 2007 | US 2009/0156759 A1<br>entire text, all drawings<br>EP 1956036 A1 | |
| WO 2007/069529 A1 | 21 June 2007 | EP 1961731 A1<br>entire text, all drawings<br>CN 101331107 A | |
| WO 2006/043491 A1 | 27 April 2006 | US 2007/0260084 A1<br>entire text, all drawings<br>EP 1803704 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006025478 A
- WO 2007072705 A
- WO 2004105696 A
- WO 200734669 A
- WO 200760893 A
- WO 200760984 A
- WO 200769462 A
- WO 200769513 A
- WO 200769514 A
- WO 200772728 A
- WO 2005123638 A
- WO 200774692 A
- WO 200788782 A
- WO 20061256 A
- WO 20061257 A
- WO 20066566 A
- WO 20066568 A
- WO 20066585 A
- WO 20066588 A
- WO 200641075 A
- WO 200643491 A
- WO 200769529 A
- WO 200769531 A
- WO 200772705 A
- WO 2005783 A
- WO 2007114130 A
- WO 200355840 A
- WO 2011105442 A
- WO 200667982 A
- JP 2021163793 A